(19) **Europäisches Patentamt / European Patent Office / Office européen des brevets**

(11) **EP 4 260 873 A1**

(12) **EUROPEAN PATENT APPLICATION**
published in accordance with Art. 153(4) EPC

(43) Date of publication:
**18.10.2023 Bulletin 2023/42**

(21) Application number: **21906560.4**

(22) Date of filing: **13.12.2021**

(51) International Patent Classification (IPC):
*A61K 45/06* (2006.01)  *A61K 31/40* (2006.01)
*A61K 31/403* (2006.01)  *A61K 31/404* (2006.01)
*A61K 31/4162* (2006.01)  *A61K 31/44* (2006.01)
*A61K 31/4439* (2006.01)  *A61K 31/47* (2006.01)
*A61K 31/496* (2006.01)  *A61K 31/4985* (2006.01)
*A61K 31/506* (2006.01)  *A61K 31/513* (2006.01)
*A61K 31/519* (2006.01)  *A61K 31/522* (2006.01)
*A61K 31/713* (2006.01)  *A61K 48/00* (2006.01)
*A61P 35/00* (2006.01)  *A61P 43/00* (2006.01)
*C12N 9/99* (2006.01)  *C12N 15/11* (2006.01)
*C12N 15/113* (2010.01)

(52) Cooperative Patent Classification (CPC):
**A61K 31/40; A61K 31/403; A61K 31/404;
A61K 31/4162; A61K 31/44; A61K 31/4439;
A61K 31/47; A61K 31/496; A61K 31/4985;
A61K 31/506; A61K 31/513; A61K 31/519;
A61K 31/522; A61K 31/713; A61K 45/06;**    (Cont.)

(86) International application number:
**PCT/JP2021/045772**

(87) International publication number:
**WO 2022/131198 (23.06.2022 Gazette 2022/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.12.2020 JP 2020206499**

(71) Applicant: **Saitama Medical University
Iruma-gun, Saitama 350-0495 (JP)**

(72) Inventors:
• **INOUE, Satoshi
Iruma-gun, Saitama 350-0495 (JP)**
• **IKEDA, Kazuhiro
Iruma-gun, Saitama 350-0495 (JP)**
• **INOUE, Kuniko
Iruma-gun, Saitama 350-0495 (JP)**
• **KAMADA, Shuhei
Iruma-gun, Saitama 350-0495 (JP)**
• **KAWAKAMI, Satoru
Iruma-gun, Saitama 350-0495 (JP)**

(74) Representative: **Graf von Stosch
Patentanwaltsgesellschaft mbH
Prinzregentenstraße 22
80538 München (DE)**

Remarks:
The complete document including Reference Table(s) and the Sequence Listing(s) can be downloaded from the EPO website

(54) **COMBINATION DRUG FOR TREATING KIDNEY CANCER AND THERAPEUTIC EFFECT ENHANCER FOR TYROSINE KINASE INHIBITOR**

(57)    The present invention relates a combination drug for treating a renal cancer and a potentiator for therapeutic effects of a tyrosine kinase inhibitor. The combination drug includes a combination of a tyrosine kinase inhibitor, and a dipeptidyl peptidase-4 inhibitor. The potentiator includes a dipeptidyl peptidase-4 inhibitor. The potentiator enhances therapeutic effects of a tyrosine kinase inhibitor on a renal cancer.

EP 4 260 873 A1

FIG.6a

(52) Cooperative Patent Classification (CPC): (Cont.)
   **A61K 48/00; A61P 35/00; A61P 43/00;**
   **C12N 9/99; C12N 15/11; C12N 15/113**

**Description**

Technical Field

The present invention relates to a combination drug for treating a renal cancer and a potentiator for therapeutic effects of a tyrosine kinase inhibitor on a renal cancer.

Background Art

Renal cell carcinoma (RCC) is one of the most common tumors among varieties of a renal cancer for adults, and the RCC incidence is currently over 400,000 cases worldwide (see NPLs 1 to 2). Five-year survival of RCC patients who undergo radical excision is promising, generally 90% or greater. Conversely, 5-year survival of metastatic RCC patients is from 10% to 20% (see NPL 3). In recent years, immune checkpoint inhibitors and molecularly targeted agents including a tyrosine kinase inhibitor (TKI) etc. have been used for treatment, however, further improvement in therapeutic effects is desired to extend the mean survival of RCC patients.

From the recent development of cancer research, it has been made clear that cancer stem-like cells (CSC) have capabilities of indefinite cell divisions and self-regeneration (see NPL 4). In CSC, cancer stem-like cell related genes, such as CD44, CD133, OCT3/4, aldehyde dehydrogenase 1 (ALDH1), CXC-chemokine receptor 4 (CXCR4), and the like, are expressed, and the above-mentioned genes are often associated with progression of tumors or resistance to cancer treatment. Interleukin-6 (IL6) is a hyperactive cytokine. It has been known that treatment using a tyrosine kinase inhibitor (TKI) induces secretion of IL6 and activation of the pathway of the AKT-mammalian target of rapamycin (mTOR), and overexpression of IL6 may be a pathological factor for TKI resistance of RCC (see NPL 5).

Dipeptidyl peptidase IV (dipeptidyl peptidase-4, DPP4)/CD26 is integral membrane glycoprotein and also serine exopeptidase. DPP4 (CD26) has been recently indicated as a protein associated with formation of cancer stem-like cells of solid tumors including multiple myeloma.

Citation List

[Non-Patent Literature]

[NPL 1] Siegel, R. L., Miller, K. D. & Jemal, A. Cancer statistics, 2018. CA Cancer J. Clin. 68, 7-30 (2018)
[NPL 2] Bray, F. et. al. Global cancer statistics 2018: GLOBOCAN estimates of incidence and mortality worldwide for 36 cancers in 185 countries. CA Cancer J. Clin. 68, 394-424 (2018)
[NPL 3] Ghatalia, P, Zibelman, M, Geynisman, D. M., Plimack, E. R. Checkpoint Inhibitors for the Treatment of Renal Cell Carcinoma. Current treatment options in oncology 18, 7 (2017)
[NPL 4] Clevers, H. The cancer stem cell: premises, promises and challenges. Nat. Med. 17, 313-319 (2011)
[NPL 5] Ishibashi, K. et al. Overriding TKI resistance of renal cell carcinoma by combination therapy with IL-6 receptor blockade. Oncotarget 8, 55230-55245 (2017)

Summary of Invention

Technical Problem

The present invention aims to solve the above-described various problems existing in the related art and to achieve the following object. Specifically, an object of the present invention is to provide a combination drug for treating a renal cancer, which has excellent therapeutic effects on a renal cancer, and a potentiator for therapeutic effects of a tyrosine kinase inhibitor, which can enhance therapeutic effects of a tyrosine kinase inhibitor on a renal cancer.

Solution to Problem

As a result of research diligently conducted by the present inventors to achieve the above-described object, the present inventors have found that use of a dipeptidyl peptidase-4 inhibitor and a tyrosine kinase inhibitor in combination can enhance therapeutic effects of a tyrosine kinase inhibitor on a renal cancer including a renal cancer having resistance to the tyrosine kinase inhibitor.

Means for solving the above-described problems are as follows.

<1> A combination drug for treating a renal cancer, the combination drug including:

> a combination of
> a tyrosine kinase inhibitor, and
> a dipeptidyl peptidase-4 inhibitor.

<2> A method of treating a renal cancer, the method including
administering the combination drug according to <1> to a subject.
<3> A potentiator for therapeutic effects of a tyrosine kinase inhibitor, the potentiator including:
a dipeptidyl peptidase-4 inhibitor,
wherein the potentiator enhances therapeutic effects of a tyrosine kinase inhibitor on a renal cancer.
<4> A method of enhancing therapeutic effects of a tyrosine kinase inhibitor on a renal cancer, the method including:
administering the potentiator according to <3> to a subject.

Advantageous Effects of Invention

According to the present invention, the above-described various problems existing in the related art can be solved; a combination drug for treating a renal cancer, which has excellent therapeutic effects on a renal cancer, and a potentiator for therapeutic effects of a tyrosine kinase inhibitor, which can enhance therapeutic effects of a tyrosine kinase inhibitor on a renal cancer, can be provided.

Brief Description of Drawings

[Fig. 1a] Fig. 1a is a diagram depicting the correlation analysis result between the expression level of DPP4 and the expression level of the cancer stem-like cell-related gene (CD44) in RCC patient-derived spheroids (co-expression of DPP4 and the cancer stem-like cell-related gene in the patient-derived RCC spheroids).
[Fig. 1b] Fig. 1b is a diagram depicting the correlation analysis result between the expression level of DPP4 and the expression level of the cancer stem-like cell-related gene (CD133) in RCC patient-derived spheroids (co-expression of DPP4 and the cancer stem-like cell-related gene in the patient-derived RCC spheroids).
[Fig. 1c] Fig. 1c is a diagram depicting the correlation analysis result between the expression level of DPP4 and the expression level of the cancer stem-like cell-related gene (OCT3/4) in RCC patient-derived spheroids (co-expression of DPP4 and the cancer stem-like cell-related gene in the patient-derived RCC spheroids).
[Fig. 1d] Fig. 1d is a diagram depicting the correlation analysis result between the expression level of DPP4 and the expression level of the cancer stem-like cell-related gene (CXCR4) in RCC patient-derived spheroids (co-expression of DPP4 and the cancer stem-like cell-related gene in the patient-derived RCC spheroids).
[Fig. 1e] Fig. 1e is a diagram depicting the correlation analysis result between the expression level of DPP4 and the expression level of the cancer stem-like cell-related gene (ALDH1A1) in RCC patient-derived spheroids (co-expression of DPP4 and the cancer stem-like cell-related gene in the patient-derived RCC spheroids).
[Fig. 1f] Fig. 1f is a diagram depicting the correlation analysis result between the expression level of DPP4 and the expression level of the cancer stem-like cell-related gene (ALDH1A2) in RCC patient-derived spheroids (co-expression of DPP4 and the cancer stem-like cell-related gene in the patient-derived RCC spheroids).
[Fig. 1g] Fig. 1g is a diagram depicting the correlation analysis result between the expression level of DPP4 and the expression level of the cancer stem-like cell-related gene (ALDH1A3) in RCC patient-derived spheroids (co-expression of DPP4 and the cancer stem-like cell-related gene in the patient-derived RCC spheroids).
[Fig. 1h] Fig. 1h is a diagram depicting the correlation analysis result between the expression level of DPP4 and the expression level of the cancer stem-like cell-related gene (IL6) in RCC patient-derived spheroids (co-expression of DPP4 and the cancer stem-like cell-related gene in the RCC patient-derived spheroids).
[Fig. 2a] Fig. 2a is a diagram depicting typical morphological characteristics, and the results of hematoxylin and eosin (H/E) staining and DPP4 immunohistochemical staining of high DPP4 expression primary RCC tumors and spheroids thereof from RCC-A and RCC-B patients, where all panels are in the same magnification, and the scale bar indicates 50 μm.
[Fig. 2b] Fig. 2b is a diagram depicting the results demonstrating the effect obtained by combining SUN and the DPP4 inhibitor SITA on a cell survival rate of the RCC-A spheroids.
[Fig. 2c] Fig. 2c is a diagram depicting the results demonstrating the effect obtained by combining SUN and the DPP4 inhibitor SITA on a cell survival rate of the RCC-B spheroids.
[Fig. 2d] Fig. 2d is a diagram depicting the results demonstrating the expression level of DPP4 in the RCC-A spheroids treated with control siRNA (siControl) and the expression levels of DPP4 in the RCC-A spheroids treated with DPP4-specific siRNA (siDPP4 #1 and #2).

[Fig. 2e] Fig. 2e is a diagram depicting the results demonstrating the expression level of DPP4 in the RCC-B spheroids treated with control siRNA (siControl) and the expression levels of DPP4 in the RCC-B spheroids treated with DPP4-specific siRNA (siDPP4 #1 and #2).

[Fig. 2f] Fig. 2f is a diagram depicting the results demonstrating the effect of DPP4-specific siRNA on a cell survival rate of the RCC-A spheroids treated with SUN.

[Fig. 2g] Fig. 2g is a diagram depicting the results demonstrating the effect of DPP4-specific siRNA on a cell survival rate of the RCC-B spheroids treated with SUN.

[Fig. 3a] Fig. 3a is a diagram depicting the results comparing the mRNA expression level of DPP4 in ACHN-R cells and the mRNA expression level of DPP4 in the parent cell line thereof (ACHN cells).

[Fig. 3b] Fig. 3b is a diagram depicting the results comparing the mRNA expression level of the CSC-related gene (CD44) in ACHN-R cells and the mRNA expression level of the CSC-related gene (CD44) in the parent cell line thereof (ACHN cells).

[Fig. 3c] Fig. 3c is a diagram depicting the results comparing the mRNA expression level of the CSC-related gene (CD133) in ACHN-R cells and the mRNA expression level of the CSC-related gene (CD133) in the parent cell line thereof (ACHN cells).

[Fig. 3d] Fig. 3d is a diagram depicting the results comparing the mRNA expression level of the CSC-related gene (OCT3/4) in ACHN-R cells and the mRNA expression level of the CSC-related gene (OCT3/4) in the parent cell line thereof (ACHN cells).

[Fig. 3e] Fig. 3e is a diagram depicting the results comparing the mRNA expression level of the CSC-related gene (ALDH1A1) in ACHN-R cells and the mRNA expression level of the CSC-related gene (ALDH1A1) in the parent cell line thereof (ACHN cells).

[Fig. 3f] Fig. 3f is a diagram depicting the results comparing the mRNA expression level of the CSC-related gene (ALDH1A2) in ACHN-R cells and the mRNA expression level of the CSC-related gene (ALDH1A2) in the parent cell line thereof (ACHN cells).

[Fig. 3g] Fig. 3g is a diagram depicting the results comparing the mRNA expression level of the CSC-related gene (ALDH1A3) in ACHN-R cells and the mRNA expression level of the CSC-related gene (ALDH1A3) in the parent cell line thereof (ACHN cells).

[Fig. 3h] Fig. 3h is a diagram depicting the results comparing the mRNA expression level of the CSC-related gene (IL6) in ACHN-R cells and the mRNA expression level of the CSC-related gene (IL6) in the parent cell line thereof (ACHN cells).

[Fig. 3i] Fig. 3i is a diagram depicting the results comparing the mRNA expression level of the DPP4 in 769-P-R cells and the mRNA expression level of DPP4 in the parent cell line thereof (769-P cells) .

[Fig. 3j] Fig. 3j is a diagram depicting the results comparing the mRNA expression level of the CSC-related gene (CD44) in 769-P-R cells and the mRNA expression level of a CSC-related gene (CD44) in the parent cell line thereof (769-P cells).

[Fig. 3k] Fig. 3k is a diagram depicting the results comparing the mRNA expression level of the CSC-related gene (CD133) in 769-P-R cells and the mRNA expression level of a CSC-related gene (CD133) in the parent cell line thereof (769-P cells).

[Fig. 3l] Fig. 3l is a diagram depicting the results comparing the mRNA expression level of the CSC-related gene (OCT3/4) in 769-P-R cells and the mRNA expression level of a CSC-related gene (OCT3/4) in the parent cell line thereof (769-P cells).

[Fig. 3m] Fig. 3m is a diagram depicting the results comparing the mRNA expression level of the CSC-related gene (ALDH1A1) in 769-P-R cells and the mRNA expression level of a CSC-related gene (ALDH1A1) in the parent cell line thereof (769-P cells).

[Fig. 3n] Fig. 3n is a diagram depicting the results comparing the mRNA expression level of the CSC-related gene (ALDH1A2) in 769-P-R cells and the mRNA expression level of a CSC-related gene (ALDH1A2) in the parent cell line thereof (769-P cells).

[Fig. 3o] Fig. 3o is a diagram depicting the results comparing the mRNA expression level of the CSC-related gene (ALDH1A3) in 769-P-R cells and the mRNA expression level of the CSC-related gene (ALDH1A3) in the parent cell line thereof (769-P cells).

[Fig. 3p] Fig. 3p is a diagram depicting the results comparing the mRNA expression level of the CSC-related gene (IL6) in 769-P-R cells and the mRNA expression level of the CSC-related gene (IL6) in the parent cell line thereof (769-P cells).

[Fig. 4a] Fig. 4a is a diagram depicting the results demonstrating the dose-response effect of sitagliptin (SITA) on three-dimensional spheroid proliferation in ACHN-R cells and the dose-response effect of sitagliptin (SITA) on three-dimensional spheroid proliferation in the parent cell line thereof.

[Fig. 4b] Fig. 4b is a diagram depicting the results demonstrating the dose-response effect of sitagliptin (SITA) on three-dimensional spheroid proliferation in 769-P-R cells and the dose-response effect of sitagliptin (SITA) on three-dimensional spheroid proliferation in the parent cell line thereof.

[Fig. 4c] Fig. 4c is a diagram depicting the results demonstrating the effect obtained by combining SITA and SUN in the 3D culture of ACHN cells.

[Fig. 4d] Fig. 4d is a diagram depicting the results demonstrating the effect obtained by combining SITA and SUN in 3D culture of ACHN-R cells.

[Fig. 4e] Fig. 4e is a diagram depicting the results demonstrating the effect obtained by combining SITA and SUN in 3D culture of 769-P cells.

[Fig. 4f] Fig. 4f is a diagram depicting the results demonstrating the effect obtained by combining SITA and SUN in 3D culture of 769-P-R cells.

[Fig. 4g] Fig. 4g is a diagram depicting the results demonstrating how DPP4 knockdown by siRNA affects therapeutic effects of SUN in 3D culture of ACHN cells.

[Fig. 4h] Fig. 4h is a diagram depicting the results demonstrating how DPP4 knockdown by siRNA affects therapeutic effects of SUN in 3D culture of ACHN-R cells.

[Fig. 4i] Fig. 4i is a diagram depicting the results demonstrating how DPP4 knockdown by siRNA affects therapeutic effects of SUN in 3D culture of 769-P cells.

[Fig. 4j] Fig. 4j is a diagram depicting the results demonstrating how DPP4 knockdown by siRNA affects therapeutic effects of SUN in 3D culture of 769-P-R cells.

[Fig. 4k] Fig. 4k is a diagram depicting the results demonstrating how the SITA treatment affects the IL6 mRNA level in 3D culture of ACHN-R cells.

[Fig. 4l] Fig. 4l is a diagram depicting the results demonstrating how the SITA treatment affects the IL6 mRNA level in 3D culture of 769-P-R cells.

[Fig. 4m] Fig. 4m is a diagram depicting the results demonstrating how overexpression of DPP4 affects the cell survival rate of the RCC spheroids (ACHN) treated with SUN.

[Fig. 4n] Fig. 4n is a diagram depicting the results demonstrating how overexpression of DPP4 affects the cell survival rate of the RCC spheroids (769-P) treated with SUN.

[Fig. 5a] Fig. 5a is a diagram depicting the results demonstrating how addition of RA to the ACHN-R cells affects suppression of the DPP4 expression by the ALDH1 inhibitor, disulfiram.

[Fig. 5b] Fig. 5b is a diagram depicting the results demonstrating how addition of RA to the 769-P-R cells affects suppression of the DPP4 expression by the ALDH1 inhibitor, disulfiram.

[Fig. 5c] Fig. 5c is a schematic view illustrating the location of a functional retinoic acid response element (RARE) and the location of the reference region near the DPP4 gene locus on the chromosome 2q24.

[Fig. 5d] Fig. 5d is a diagram depicting the results to confirm recruitment of the retinoic acid receptor $\alpha$ (RAR$\alpha$) to RARE on the DPP4 promoter of the ACHN-R cells.

[Fig. 5e] Fig. 5e is a diagram depicting the results to confirm recruitment of the retinoic acid receptor $\alpha$ (RAR$\alpha$) to RARE on the DPP4 promoter of the 769-P-R cells.

[Fig. 5f] Fig. 5f is a diagram depicting the results to confirm recruitment of retinoid X receptor $\alpha$ (RXRa) to RARE on the DPP4 promoter of the ACHN-R cells.

[Fig. 5g] Fig. 5g is a diagram depicting the results to confirm recruitment of retinoid X receptor $\alpha$ (RXRa) to RARE on the DPP4 promoter of the 769-P-R cells.

[Fig. 5h] Fig. 5h is a diagram depicting the results demonstrating how the RA treatment of 293T cells affects the RARE sequence.

[Fig. 6a] Fig. 6a is a diagram depicting the tumor proliferation curves of the ACHN/SUN group, ACHN-R/SUN group, and ACHN-R/SUN+SITA group.

[Fig. 6b] Fig. 6b is a diagram depicting the measurement results of the tumor weights of the ACHN/SUN group, ACHN-R/SUN group, and ACHN-R/SUN+SITA group.

[Fig. 6c] Fig. 6c is a diagram depicting the measurement results of the weights of the ACHN/SUN group, ACHN-R/SUN group, and ACHN-R/SUN+SITA group.

[Fig. 6d] Fig. 6d is a diagram depicting a typical photograph of the xenograft nude mouse model of the ACHN/SUN group on the 13th day.

[Fig. 6e] Fig. 6e is a diagram depicting a typical photograph of the xenograft nude mouse model of the ACHN-R/SUN group on the 13th day.

[Fig. 6f] Fig. 6d is a diagram depicting a typical photograph of the xenograft nude mouse model of the ACHN-R/SUN+SITA group on the 13th day.

[Fig. 6g] Fig. 6g is diagram depicting the results obtained by Ki67 immunohistochemical staining the tumors excised from each of the ACHN/SUN group, ACHN-R/SUN group, and ACHN-R/SUN+SITA group, and determining the ratio of the Ki67 positive cells (Ki67 index).

[Fig. 6h] Fig. 6h is a diagram depicting the results of the mRNA levels of DPP4 in the ACHN/SUN group, ACHN-R/SUN group, and ACHN-R/SUN+SITA group.

[Fig. 6i] Fig. 6i is a diagram depicting the results of the mRNA levels of IL6 in the ACHN/SUN group, ACHN-R/SUN group, and ACHN-R/SUN+SITA group.

[Fig. 7a] Fig. 7a is a diagram depicting the results of the Kaplan-Meier 10-year overall survival (OS) determined from the initial consultation for 73 cases of the patients subjected to the stratified TKI treatment depending on the existence or nonexistence of Type 2 diabetes mellitus (T2DM) and administration or no administration of the DPP4 inhibitor (DPP4i).

[Fig. 7b] Fig. 7b is a diagram depicting the results of waterfall plot analysis of the maximum tumor changes of the 73 RCC patients subjected to the stratified TKI treatment depending on the existence or nonexistence of T2DM and administration or no administration of the DPP4i.

[Fig. 7c] Fig. 7c is a diagram depicting the results of the box plot analysis of the maximum tumor changes of the 73 RCC patients subjected to the stratified TKI treatment depending on the existence or nonexistence of T2DM and administration or no administration of DPP4i.

[Fig. 7d] Fig. 7d is a diagram depicting the results of the Kaplan-Meier analysis of 10-year overall survival in association with the DPP4 expression and prognosis.

[Fig. 7e] Fig. 7e is a diagram depicting the results of the Kaplan-Meier analysis of 10-year overall survival, for only the 31 non-T2DM cases, in association with the DPP4 expression and prognosis.

[Fig. 7f] Fig. 7f is a diagram depicting the results of Kaplan-Meier analysis of 10-year overall survival, for only the 18 T2DM cases, in association with the DPP4 expression and prognosis.

[Fig. 7g] Fig. 7g is a schematic view of the ALDH1/retinoic acid/DPP4 system (axis) in the renal cancer stem-like cell of high DPP4 expression.

Description of Embodiments

(Combination drug)

The combination drug of the present invention is a combination drug for treating a renal cancer. The combination drug includes a combination of a tyrosine kinase inhibitor and a dipeptidyl peptidase-4 inhibitor, and may further include other ingredients as necessary.

In the present invention, the term "treating" means improving or reducing symptoms of a cancer, and/or preventing any progress of the disease.

<Renal cancer>

The renal cancer is not particularly limited, and may be appropriately selected according to the intended purpose. Examples of the renal cancer include renal cell carcinoma, a renal pelvic cancer, and the like. The renal cancer may be a metastatic renal cancer, or may not be a metastatic renal cancer. Moreover, the renal cancer may be a renal cancer having resistance (may be referred to as "invulnerability") to a tyrosine kinase inhibitor, or may be a renal cancer not having resistance to the tyrosine kinase inhibitor. The combination drug of the present invention can be suitably used for the renal cancer having resistance to the tyrosine kinase inhibitor.

<Tyrosine kinase inhibitor>

The tyrosine kinase inhibitor is not particularly limited, provided that the tyrosine kinase inhibitor can inhibit enzymatic activities of tyrosine kinase, or can suppress expression of tyrosine kinase. The tyrosine kinase inhibitor may be appropriately selected according to the intended purpose. Examples of the tyrosine kinase inhibitor include sunitinib, sorafenib, axitinib, pazopanib, cabozantinib, lenvatinib, and the like. The above-listed examples may be used alone or in combination.

As the tyrosine kinase inhibitor, a commercially available product may be used, or an appropriately synthesized product may be used.

<Dipeptidyl peptidase-4 inhibitor>

The dipeptidyl peptidase-4 inhibitor is not particularly limited, provided that the dipeptidyl peptidase-4 inhibitor can inhibit enzymatic activities of dipeptidyl peptidase-4 or can suppress expression of dipeptidyl peptidase-4. The dipeptidyl peptidase-4 inhibitor may be appropriately selected according to the intended purpose. Examples of the dipeptidyl peptidase-4 inhibitor include sitagliptin, linagliptin, alogliptin, teneligliptin, anagliptin, vildagliptin, saxagliptin, trelagliptin, omarigliptin, gemigliptin, evogliptin, gosogliptin, dipeptidyl peptidase-4 expression inhibitors, and the like. The above-listed examples may be used alone or in combination.

As the dipeptidyl peptidase-4 inhibitor, a commercially available product may be used, or an appropriately synthesized product may be used.

<<Dipeptidyl peptidase-4 expression inhibitor>>

The dipeptidyl peptidase-4 expression inhibitor is not particularly limited, provided that the dipeptidyl peptidase-4 expression inhibitor can suppress expression of dipeptidyl peptidase-4. The dipeptidyl peptidase-4 expression inhibitor may be appropriately selected according to the intended purpose. Examples of the dipeptidyl peptidase-4 expression inhibitor include double-stranded nucleic acid molecules inhibiting expression of a dipeptidyl peptidase-4 gene (may be referred to as "double-stranded nucleic acid molecule(s)" hereinafter), DNA including a base sequence encoding the double-stranded nucleic acid molecule (may be referred to as "DNA"), vectors including the DNA (may be referred to as "vector(s)" hereinafter), and the like. The above-listed examples may be used alone or in combination.

-Double-stranded nucleic acid molecule-

The double-stranded nucleic acid molecule is not particularly limited, provided that the double-stranded nucleic acid molecule can suppress expression of a dipeptidyl peptidase-4 gene. The double-stranded nucleic acid molecule may be appropriately selected according to the intended purpose. Examples of the double-stranded nucleic acid molecule include double-stranded nucleic acid molecules each including (a) a sense strand including a base sequence corresponding to a target sequence composed of a base sequence of SEQ ID NO: 1 or SEQ ID NO: 4, and (b) an antisense strand that constitutes a double stranded nucleic acid with the sense strand of (a), and includes a base sequence complimentary to the sense strand of (a), and the like. The above-listed examples may be used alone or in combination.

In the present invention, the term "double-stranded nucleic acid molecule" encompasses a double-stranded nucleic acid molecule in which a sense strand and an antisense strand are hybridized.

In the present invention, mRNA of the dipeptidyl peptidase-4 gene is a target of the double-stranded nucleic acid molecule so that the double-stranded nucleic acid molecule suppresses expression of the dipeptidyl peptidase-4 gene. Accordingly, the dipeptidyl peptidase-4 gene may be referred to as a "target gene" of the double-stranded nucleic acid molecule in the present specification.

--Sense strand and antisense strand--

The double-stranded nucleic acid molecule preferably includes (a) a sense strand including a base sequence corresponding to a target sequence composed of a base sequence of SEQ ID NO: 1 or SEQ ID NO: 4, and (b) an antisense strand that constitutes a double stranded nucleic acid with the sense strand of (a), and includes a base sequence complimentary to the sense strand.

The sense strand and the antisense strand may be RNA strands or chimeric RNA-DNA strands. The sense strand and the antisense strand may be hybridized to each other to form the double-stranded nucleic acid molecule.

The sense strand of the double-stranded nucleic acid molecule is not limited, as long as the sense strand includes a base sequence corresponding to the target sequence. The sense strand may further include other base sequences, or may be composed only of the base sequence corresponding to the target sequence.

Moreover, the antisense strand of the double-stranded nucleic acid molecule is not limited, as long as the antisense strand includes a base sequence complimentary to the sense strand, where the degree of the complementarity is sufficient enough for the antisense strand and the sense strand to hybridize. The antisense strand may further include other base sequences. The antisense strand preferably includes the base sequence complimentary to the sense strand in the proportion of 700 or greater, more preferably 800 or greater, further preferably 90% or greater, and particularly preferably 95% or greater.

A type of the double-stranded nucleic acid molecule is not particularly limited, and may be appropriately selected according to the intended purpose. Examples of the type of the double-stranded nucleic acid molecule include double-stranded RNA (dsRNA), double-stranded RNA-DNA chimeras, and the like.

In the present specification, the term "double-stranded RNA" encompasses a double-stranded nucleic acid molecule, in which both a sense strand and antisense strand are composed of RNA sequences; the term "double-stranded RNA-DNA chimera" encompasses a double-stranded nucleic acid molecule, in which both the sense strand and antisense strand are composed of chimeric RNA-DNA sequences.

The double-stranded RNA or double-stranded RNA-DNA chimera is preferably small interfering RNA (siRNA) or chimeric siRNA, more preferably siRNA.

In the present specification, siRNA is a small-molecule double-stranded RNA having a length of 18 bases to 29 bases, and has a function of cleaving a target RNA having a sequence complimentary to the antisense strand (guide strand) of the siRNA to suppress expression of the target RNA.

The terminus structure of the siRNA is not particularly limited, provided that the siRNA includes the above-described sense strand and antisense strand, and is capable of suppressing expression of a target RNA. The terminus structure of the siRNA may be appropriately selected according to the intended purpose. For example, the siRNA may have blunt ends, or sticky ends (overhangs). Among the above-listed examples, the siRNA preferably has a structure where the 3' end of each chain is stuck out by 2 bases to 6 bases, more preferably a structure where the 3' end of each chain is stuck out by 2 bases.

Moreover, the chimeric siRNA is a small-molecule double-stranded RNA-DNA chimera having a length of 18 bases to 29 bases, where part of the RNA sequence of siRNA is converted to DNA. Among the above-described chimeric siRNAs, the chimeric siRNA is preferably a small-molecule double-stranded RNA-DNA chimera having a length of 21 bases to 23 bases, where the bases within the 8 bases of the 3' side of the sense strand of siRNA or the bases within the 6 bases of the 5' side of the sense strand of siRNA are converted to DNA. Like the siRNA, the chimeric siRNA has a function of suppressing expression of a target gene. Note that, the chimeric siRNA also includes an embodiment of the chimeric siRNA, in which part of the sequence converted to DNA is again converted to RNA.

Similar to the siRNA, the terminus structure of the chimeric siRNA is not particularly limited, and may be appropriately selected according to the intended purpose. For example, the chimeric siRNA may have blunt ends, or sticky ends (overhangs).

Specific examples of the siRNA include the following.

Examples of the siRNA whose target sequence is the base sequence of the SEQ ID NO: 1 include siRNA composed of the sense strand of SEQ ID NO: 2 below and the antisense strand of SEQ ID NO: 3 below.

Sense strand

5'-GGAGGGUACGUAACCUCAAUG-3' (SEQ ID NO: 2)

Antisense strand

5'-UUGAGGUUACGUACCCUCCAU-3' (SEQ ID NO: 3)

Moreover, Examples of the siRNA whose target sequence is the base sequence of the SEQ ID NO: 4 include siRNA composed of the sense strand of SEQ ID NO: 5 below and the antisense strand of SEQ ID NO: 6 below.

Sense strand

5'-CAGUCGCAAAACUUACACUCU-3' (SEQ ID NO: 5)

Antisense strand

5'-AGUGUAAGUUUUGCGACUGUC-3' (SEQ ID NO: 6)

Moreover, the double-stranded RNA may be short hairpin RNA (shRNA). The shRNA is single-stranded RNA including a dsRNA region of about 18 bases to about 29 bases, and a loop region of about 3 bases to about 9 bases. As the shRNA is expressed in vivo, the shRNA forms base pairs to construct a hairpin double-stranded RNA. Then, the shRNA is cleaved by Dicer (RNase III enzyme) to become siRNA to function to suppress expression of target RNA. Similar to the siRNA and double-stranded RNA-DNA chimera, the terminus structure of the shRNA is not particularly limited, and may be appropriately selected according to the intended purpose. For example, the shRNA may have blunt ends, or sticky ends (overhangs).

Moreover, the double-stranded nucleic acid molecule may be appropriately modified according to the intended purpose. For example, 2'-O-methylation modification, phosphorothioate (S-) modification, locked nucleic acid (LNA) modification or the like may be performed on the double-stranded nucleic acid molecule to impart resistance to nucleolytic enzymes (nuclease) to improve stability in a liquid culture medium or in vivo. For example, moreover, the 5' end or 3' end of the sense strand of the double-stranded nucleic acid molecule may be modified with nanoparticles, cholesterol, cell-penetrating peptides, or the like to increase transfer efficiency to cells. Note that, the method of modifying the double-stranded nucleic acid molecule to achieve any of the above-mentioned modifications is not particularly limited, and may be appropriately selected from methods known in the related art.

A method of acquiring the double-stranded nucleic acid molecule is not particularly limited, and may be produced according to any method selected from the methods known in the related art.

For example, the siRNA can be produced by chemically synthesizing two single-stranded RNA sequences each having a length of 18 bases to 29 bases corresponding to the predetermined sense strand and the predetermined antisense strand, respectively, using a known automatic DNA/RNA synthesis device, etc., and annealing the synthesized two single-stranded RNA sequences. Moreover, a commercial product of an annealed double-stranded siRNA may be acquired. Alternatively, the siRNA may be acquired by commissioning a contract siRNA synthesis service provider to synthesize the siRNA. Moreover, a suitable siRNA-expression vector, such as the below-described vector of the present invention, is constructed, followed by transferring the expression vector into a cell to produce siRNA utilizing a reaction within the cell.

Moreover, the chimeric siRNA can be produced, for example, by chemically synthesizing both a sense strand and an antisense strand, which are chimeric nucleic acid molecules, and annealing the sense strand and the antisense strand to produce a chimeric siRNA.

-DNA-

The DNA is not particularly limited, provided that the DNA is DNA including a base sequence encoding the above-described double-stranded nucleic acid molecule. The DNA may be appropriately selected according to the intended purpose. The DNA preferably has a sequence to which a promoter sequence is linked upstream (5' side) of the base sequence encoding the double-stranded nucleic acid molecule. The promoter sequence is a sequence for controlling transcription of the double-stranded nucleic acid molecule. The promoter sequence is not particularly limited, and may be appropriately selected according to the intended purpose. Examples of the promoter sequence include: pol II-based promoters, such as CMV promoters; pol III-based promoters, such as H1 promoters, and U6 promoters; and the like.

Moreover, a terminator sequence to terminate transcription of the double-stranded nucleic acid molecule is more preferably linked downstream (3' side) of the base sequence encoding the above-described double-stranded nucleic acid molecule. The terminator sequence is not particularly limited, and may be appropriately selected according to the intended purpose.

The transcription unit including the promoter sequence, the base sequence for encoding the double-stranded nucleic acid molecule, and the terminator sequence is one of preferable embodiments of the DNA. Note that, the transcription unit may be constructed using any method known in the related art.

-Vector-

The vector is not particularly limited, provided that the vector includes the above-described DNA. The vector may be appropriately selected according to the intended purpose. Examples of the vector include plasmid vectors, viral vectors, and the like. The vector is preferably an expression vector capable of expressing the above-described double-stranded nucleic acid molecule.

A manner of the expression of the double-stranded nucleic acid molecule is not particularly limited, and may be appropriately selected according to the intended purpose. Examples of the method of expressing siRNA as the double-stranded nucleic acid molecule include: a method of expressing two strands of short single-stranded RNA (tandem type); a method of expressing a single-stranded RNA as shRNA (hairpin type); and the like.

The tandem-type siRNA-expression vector includes DNA including a DNA sequence encoding a sense strand constituting the siRNA, and a DNA sequence encoding an antisense strand constituting the siRNA, where a promoter sequence is linked upstream (5' side) of the DNA sequence encoding each strand, and a terminator sequence is linked downstream (3' side) of the DNA sequence encoding each strand.

Moreover, the hairpin-type siRNA-expression vector includes DNA, in which a DNA sequence encoding a sense strand constituting the siRNA and a DNA sequence encoding an antisense strand constituting the siRNA are aligned in the reverse direction, the sense strand DNA sequence and the antisense strand DNA sequence are connected with a loop sequence, a promoter sequence is linked upstream (5' side) of the DNA sequence encoding each strand, and a terminator sequence is linked downstream (3' side) of the DNA sequence encoding each strand.

The above-mentioned vectors can be constructed using any method known in the related art. For example, the DNA can be constructed by linking (ligating) to a cleaved site of the vector, which is cleaved in advance by a restriction enzyme.

As the DNA of the vector is introduced (transfected) into cells, the promoter is activated to generate the double-stranded nucleic acid molecule. With the tandem vector, for example, the DNA is transcribed within a cell to generate a sense strand and an antisense strand, and the generated sense strand and antisense strand are hybridized to generate siRNA. With the hairpin-type vector, the DNA is transcribed within a cell to first generate hairpin-type RNA (shRNA), followed by processing with Dicer to generate siRNA.

<Other ingredients>

Other ingredients included in the combination drug are not particularly limited, provided that such ingredients do not adversely affect the effects obtainable by the present invention. Other ingredients are appropriately selected from pharmaceutically acceptable carriers according to the intended purpose. Examples of other ingredients include additives, adjuvants, water, and the like. The above-listed examples may be used alone or in combination.

The additives or the adjuvants are not particularly limited, and may be appropriately selected according to the intended purpose. Examples of the additives or the adjuvants include bactericide, preservatives, caking additives, thickeners, adhesives, binders, colorants, stabilizers, pH regulators, buffers, tonicity agents, solvents, antioxidants, UV-blocking agents, crystal precipitation (growth) inhibitors, defoaming agents, physical property improving agents, antiseptic agents, and the like.

The amounts of other ingredients in the combination drug are not particularly limited, and may be appropriately selected according to the intended purpose.

<Use>

The combination drug may be used as an embodiment including only a combination of the tyrosine kinase inhibitor and the dipeptidyl peptidase-4 inhibitor, or as an embodiment where the combination of the tyrosine kinase inhibitor and the dipeptidyl peptidase-4 inhibitor is further combined with a drug including other substances as active ingredients.

Moreover, the combination drug may be used in the state where the combination drug is blended with a drug including other substances as active ingredients.

The combination drug may contain the tyrosine kinase inhibitor and the dipeptidyl peptidase-4 inhibitor as two separate preparations, or as one preparation (compounded preparation).

<Dosage form>

A dosage form of the combination drug is not particularly limited, and may be appropriately selected according to the intended administration method. Examples of the dosage form include oral solid preparations (e.g., pills, coated pills, granules, powder, capsules, and the like), oral liquid preparations (e.g., oral solutions, syrups, elixirs, and the like), injectable preparations (e.g., solutions, suspensions, solid preparations to be used for reconstitution, and the like), ointment, patches, gel, cream, topical powder, spray, inhalant powder, and the like.

In the case where the tyrosine kinase inhibitor and the dipeptidyl peptidase-4 inhibitor prepared as separate preparations are used as the combination drug, the dosage form is not particularly limited, and may be appropriately selected according to the intended purpose. The dosage form of the tyrosine kinase inhibitor and the dosage form of the dipeptidyl peptidase-4 inhibitor may be the same or different.

The oral solid preparation can be produced by adding, to the active ingredients, a diluent, and further adding optional additives, such as binders, disintegrants, lubricants, colorants, flavoring agents, and the like, according to a commonly known method.

Examples of the diluent include lactose, refined sugar, sodium chloride, glucose, starch, calcium carbonate, kaolin, microcrystalline cellulose, silicic acid, and the like. Examples of the binders include water, ethanol, propanol, simple syrup, glucose solutions, starch solutions, gelatin solutions, carboxy methyl cellulose, hydroxypropyl cellulose, hydroxypropyl starch, methyl cellulose, ethyl cellulose, Shellac, calcium phosphate, polyvinyl pyrrolidone, and the like. Examples of the disintegrants include dry starch, sodium alginate, agar powder, sodium hydrogen carbonate, calcium carbonate, sodium lauryl sulfate, stearic acid monoglyceride, lactose, and the like. Examples of the lubricants include refined talc, stearate, borax, polyethylene glycol, and the like. Examples of the colorants include titanium oxide, iron oxide, and the like. Examples of the flavoring agents include refined sugar, orange peel, citric acid, tartaric acid, and the like.

The oral liquid preparation can be produced, for example, by adding, to the active ingredients, additives, such as flavoring agents, buffers, stabilizers, and the like, according to a commonly known method.

Examples of the flavoring agents include refined sugar, orange peel, citric acid, tartaric acid, and the like. Examples of the buffers include sodium citrate, and the like. Examples of the stabilizers include tragacanth, gum arabic, and gelatin, and the like.

The injectable preparation for subcutaneous injection, intramuscular injection, intravenous injection, or the like can be produced, for example, by adding, to the active ingredients, a pH regulator, a buffer, a stabilizer, a tonicity agent, local anesthetic agents, and the like, according to a commonly known method.

Examples of the pH regulators and the buffers include sodium citrate, sodium acetate, sodium phosphate, and the like. Examples of the stabilizers include sodium metabisulfite, EDTA, thioglycolic acid, thiolactic acid, and the like.

Examples of the tonicity agents include sodium chloride, glucose, and the like. Examples of the local anesthetic agents include procaine hydrochloride, lidocaine hydrochloride, and the like.

The ointment can be produced, for example, by blending, with the active ingredients, a base material, a stabilizer, a wetting agent, a preservative, and the like known in the related art according to a commonly known method.

Examples of the base material include liquid paraffin, white petrolatum, white beeswax, octyldodecyl alcohol, paraffin, and the like. Examples of the preservative include methyl parahydroxybenzoate, ethyl parahydroxybenzoate, propyl parahydroxybenzoate, and the like.

The patch can be produced, for example, by applying cream, gel, paste, or the like as the ointment onto a support known in the related art according to a commonly known method.

Examples of the support include: woven fabrics and nonwoven fabrics of cotton, staple fibers, or chemical fibers; films of soft vinyl chloride, polyethylene, polyurethane, or the like; and foam sheets; and the like.

<Administration>

A method, dose, timing, and subject of administration of the combination drug are not particularly limited, and may be appropriately selected according to the intended purpose.

The method of administering the combination drug is not particularly limited. For example, either topical administration or systemic administration may be selected according to the dose foam of the combination drug, conditions of a patient, and the like. For the topical administration, for example, the combination drug can be administered by directly injecting the active ingredients of the combination drug into a suitable site (e.g., a tumor site). For the injection, a method known in the related art, such as injection by a syringe, can be appropriately adapted. For the systematic administration (e.g., oral administration, intravascular administration, and the like), moreover, a drug delivery technology known in the art is preferably appropriately applied to stably and efficiently deliver the active ingredients of the combination drug to a suitable site (e.g., a tumor site).

In the case where the tyrosine kinase inhibitor and the dipeptidyl peptidase-4 inhibitor prepared as separate preparations are used as the combination drug, the administration method of the tyrosine kinase inhibitor and the administration method of the dipeptidyl peptidase-4 inhibitor may be the same or different.

The administration dose is not particularly limited, and may be appropriately selected considering various factors, such as age, weight, body conditions of an administration subject, presence or absence of administration of a drug or pharmaceutical preparation including other substances as active ingredients, and the like.

The ratio between the dose of the tyrosine kinase inhibitor and the dose of the dipeptidyl peptidase-4 inhibitor in the combination drug is not particularly limited, and may be appropriately selected according to the intended purpose.

Moreover, the frequency of the administration is not particularly limited, and may be appropriately selected considering various factors, such as age, weight, body conditions of an administration subject, presence or absence of administration of a drug or pharmaceutical preparation including other substances as active ingredients, and the like.

The timing of the administration is not particularly limited, and may be appropriately selected according to the intended purpose.

As the combination drug, the tyrosine kinase inhibitor and the dipeptidyl peptidase-4 inhibitor may be administered at the same time, or may be administered at different timing.

When the tyrosine kinase inhibitor and the dipeptidyl peptidase-4 inhibitor are administered at different timing, the order of administering the tyrosine kinase inhibitor and the dipeptidyl peptidase-4 inhibitor is not particularly limited, and may be appropriately selected according to the intended purpose.

When the tyrosine kinase inhibitor and the dipeptidyl peptidase-4 inhibitor are administered at different timing, moreover, the interval between the administration of the tyrosine kinase inhibitor and the administration of the dipeptidyl peptidase-4 inhibitor is not particularly limited, and may be appropriately selected according to the intended purpose.

A subject for the administration is not particularly limited, and may be appropriately selected according to the intended purpose. Examples of the subject for the administration include humans, monkeys, pigs, cows, sheep, goats, dogs, cats, mice, rats, birds, and the like. Among the above-listed examples, the combination drug is particularly suitably used for humans.

(Method of treating renal cancer)

As described in the section of Examples below, the combination drug can enhance a tumor proliferation inhibition effect (enhance therapeutic effects) of a tyrosine kinase inhibitor by using a dipeptidyl peptidase-4 inhibitor in combination. Accordingly, the present invention is also related to a method of treating a renal cancer, where the method includes administering the combination drug of the present invention to a subject.

The renal cancer is not particularly limited. Examples of the renal cancer include those described in the section of the renal cancer in association with the above-described combination drug.

The method of treating a renal cancer may further use other renal cancer drugs.

(Potentiator for therapeutic effects of tyrosine kinase inhibitor)

The potentiator for therapeutic effects of a tyrosine kinase inhibitor of the present invention includes at least a dipeptidyl peptidase-4 inhibitor, and may further include other ingredients as necessary.
The potentiator for therapeutic effects of a tyrosine kinase inhibitor can enhance therapeutic effects of the tyrosine kinase inhibitor on a renal cancer.
In the present invention, the meaning of the phrase "enhance therapeutic effects" includes enhancing a tumor proliferation inhibition effect, and recovering therapeutic effects of the tyrosine kinase inhibitor against tumors having resistance to the tyrosine kinase inhibitor (overturning the resistance).

<Renal cancer>

Examples of the renal cancer include those described in the section of the renal cancer in association with the above-described combination drug.

<Dipeptidyl peptidase-4 inhibitor>

Examples of the dipeptidyl peptidase-4 inhibitor include those listed in the section of the dipeptidyl peptidase-4 inhibitor in association with the above-described combination drug.
The amount of the dipeptidyl peptidase-4 inhibitor in the potentiator for therapeutic effects of the tyrosine kinase inhibitor is not particularly limited, and may be appropriately selected according to the intended purpose. The potentiator for therapeutic effects of the tyrosine kinase inhibitor may be composed only of the dipeptidyl peptidase-4 inhibitor.

<Other ingredients>

Other ingredients included in the potentiator for therapeutic effects of the tyrosine kinase inhibitor are not particularly limited, and may be appropriately selected from pharmaceutically acceptable carriers according to the intended purpose. Examples of such ingredients include those listed in the section of other ingredients in association with the above-described combination drug. Those listed as examples may be used alone or in combination.
The amounts of other ingredients in the potentiator for therapeutic effects of the tyrosine kinase inhibitor are not particularly limited, and may be appropriately selected according to the intended purpose.

<Tyrosine kinase inhibitor>

Examples of the tyrosine kinase inhibitor, which is a target of the potentiator for therapeutic effects of the tyrosine kinase inhibitor, include those listed in the section of the tyrosine kinase inhibitor in association with the above-described combination drug.

<Use>

The potentiator for therapeutic effects of the tyrosine kinase inhibitor may be used in combination with a tyrosine kinase inhibitor, or in combination with the tyrosine kinase inhibitor and another drug including other substances as active ingredients. Moreover, the potentiator for therapeutic effects of the tyrosine kinase inhibitor may be used in the state where the potentiator is blended into a drug including a tyrosine kinase inhibitor or other substances as active ingredients.

<Dosage form>

A dosage form of the potentiator for therapeutic effects of the tyrosine kinase inhibitor is not particularly limited, and may be appropriately selected according to the intended purpose. Examples of the dosage form include those listed in the section of the dosage form of the above-described combination drug.

<Administration>

The method, dose, timing, and subject for the administration of the potentiator for therapeutic effects of the tyrosine kinase inhibitor are not particularly limited, and may be appropriately selected according to the intended purpose.

Examples thereof include those listed in the section of the administration of the above-described combination drug.

(Method of enhancing therapeutic effects of tyrosine kinase inhibitor on renal cancer)

As described in the section of Examples below, the potentiator for therapeutic effects of the tyrosine kinase inhibitor can enhance the tumor proliferation inhibition effect of the tyrosine kinase inhibitor. Therefore, the present invention is also related to a method of enhancing therapeutic effects of a tyrosine kinase inhibitor on a renal cancer, where the method includes administering the potentiator of the present invention to a subject.

The renal cancer is not particularly limited, and may be appropriately selected according to the intended purpose. Examples of the renal cancer include those listed in the section of the renal cancer in association with the above-described combination drug.

The tyrosine kinase inhibitor administered in combination with the administration of the potentiator for therapeutic effects of the tyrosine kinase inhibitor is not particularly limited, and may be appropriately selected according to the intended purpose. Examples of the tyrosine kinase inhibitor include those listed in the section of the tyrosine kinase inhibitor of the above-described combination drug.

The method of enhancing the therapeutic effects of the tyrosine kinase inhibitor on the renal cancer may further use another drug for treating a renal cancer.

[Examples]

Test Examples of the present invention will be described hereinafter, but Test Examples shall not be construed as limiting the scope of the present invention in any way.

(Test Example 1)

<Materials and methods>

<<Clinical data collection and selection of patients>>

Seventy-three (73) cases of renal cell carcinoma (RCC) patients who received a tyrosine kinase inhibitor (TKI) between 2008 and 2019 at Saitama Medical Center, Saitama Medical University, were retrospectively analyzed. Overall survival of the patients from the initial consultation, and the maximum tumor shrinkage defined by Response Evaluation Criteria in Solid Tumors (RECIST version 1.1) (Eisenhauer, E. A. et al. New response evaluation criteria in solid tumours: revised RECIST guideline (version 1.1). Eur. J. Cancer 45, 228-247 (2009)) were evaluated. For the maximum tumor shrinkage, the most recent TKI treatment was evaluated, among the evaluatable treatments. The patients who did not wish to consent with the trial, the cases where objective images could not be obtained before and after the TKI treatment, and the patients who received only immunotherapy or an mTOR inhibitor were removed from the data. If the values of clinical factors were missing, such cases were removed from statistical analysis for comparing the characteristics of the patients. This clinical analysis has been approved by the Ethics Committee of Saitama Medical Center, Saitama Medical University (No. 117, No. 2308).

<<Productions of patient-derived cells and RCC cell lines used for experiments>>

After attaining the informed consent from the patients at Saitama Medical Center, Saitama Medical University, patient-derived cells (PDC) were produced from the tumors excised from the RCC patients. The tumor samples were processed with reference to the literature (Namekawa, T, et al. ALDH1A1 in patient-derived bladder cancer spheroids activates retinoic acid signaling leading to TUBB3 overexpression and tumor progression. Int. J. Cancer 146, 1099-1113 (2019)). The protocol of the research was approved by the Ethics Committee of Saitama Medical Center, Saitama Medical University (No. 1363-IV).

ACHN and 769-P, which were human RCC cell lines, were acquired from the American Type Culture Collection (ATCC), and those authenticated by short tandem repeat (STR) analysis performed by BEX CO., LTD. were used. The ACHN and 769-P cells were cultured on a DEME medium and a PRMI medium (NACALI TESQUE, INC.), respectively, to both of which 10% of FBS, 100 U/mL of penicillin, and 100 $\mu$g/mL of streptomycin were added, using an incubator set at 37°C and 5% $CO_2$. The ACHN and 769-P cells were exposed to sunitinib (SUN) at 10 $\mu$M max., for 6 months or longer, to produce SUN resistant RCC cell lines, ACHN-R and 769-P-R, respectively.

<<Transfection of siRNA>>

siRNA (siDPP4 #1 and #2), whose target was dipeptidyl peptidase-4 (DPP4), and control siRNA (siControl) were acquired from RNAi Inc., and were introduced into the cells using the RNAiMAX reagent (Thermo Fisher Scientific K.K.) according to the instructions from the manufacturer.

Th sequences of the siRNA are presented below.

[siDPP4 #1]

-Target sequence-
5'-GGAGGGTACGTAACCTCAATG-3' (SEQ ID NO: 1)

-Sequence of double-stranded nucleic acid molecule (siRNA)-

Sense strand
5'-GGAGGGUACGUAACCUCAAUG-3' (SEQ ID NO: 2)

Antisense strand
5'-UUGAGGUUACGUACCCUCCAU-3' (SEQ ID NO: 3)

[siDPP4 #2]

-Target sequence-
5'-CAGTCGCAAAACTTACACTCT-3' (SEQ ID NO: 4)

-Sequence of double-stranded nucleic acid molecule (siRNA)-

Sense strand
5'-CAGUCGCAAAACUUACACUCU-3' (SEQ ID NO: 5)

Antisense strand
5'-AGUGUAAGUUUUGCGACUGUC-3' (SEQ ID NO: 6)

[siControl]

-Sequence of double-stranded nucleic acid molecule (siRNA)-

Sense strand
5'-GUACCGCACGUCAUUCGUAUC-3' (SEQ ID NO: 7)

Antisense strand
5'-UACGAAUGACGUGCGGUACGU-3' (SEQ ID NO: 8)

<<Cell survival rate assay on spheroid culture>>

A cell survival rate of the cells 3 days after the drug treatment and siRNA transfection was evaluated by CellTiter-Glo 3D Assay (Promega K.K.).

<<Synthesis of cDNA and quantitative reverse transcription PCR>>

Extraction of RNA, synthesis of cDNA, and quantitative reverse-transcription polymerase chain reaction (qRT-PCR) were carried out according to the methods disclosed in "Namekawa, T, et al. ALDH1A1 in patient-derived bladder cancer spheroids activates retinoic acid signaling leading to TUBB3 overexpression and tumor progression. Int. J. Cancer 146, 1099-1113 (2019)."

The primers used for the qRT-PCR are as follows.

[DPP4]

Forward
5'-CCAAACGGCACTTTTTTAGCA-3' (SEQ ID NO: 9)

Reverse
5'-GAGTATTCAATAAGTGGGACTTCTGTGT-3' (SEQ ID NO: 10)

[CD44]

Forward
5'-GTGATGGCACCCGCTATG-3' (SEQ ID NO: 11)

Reverse
5'-ACTGTCTTCGTCTGGGATGG-3' (SEQ ID NO: 12)

[CD133]

Forward
5'-CAGAGTACAAACGCCAAACCA-3' (SEQ ID NO: 13)

Reverse
5'-AAATCACGATGAGGGTCAGC-3' (SEQ ID NO: 14)

[OCT3/4]

Forward
5'-TTCAGCCAAACGACCATCTG-3' (SEQ ID NO: 15)

Reverse
5'-CACGAGGGTTTCTGCTTTGC-3' (SEQ ID NO: 16)

[CXCR4]

Forward
5'-GCATGACGGACAAGTACAGGCT-3' (SEQ ID NO: 17)

Reverse
5'-AAAGTACCAGTTTGCCACGGC-3' (SEQ ID NO: 18)

[ALDH1A1]

Forward
5'-CGCAAGACAGGCTTTTCAGAT-3' (SEQ ID NO: 19)

Reverse
5'-CCCTCTCGGAAGCATCCA-3' (SEQ ID NO: 20)

[ALDH1A2]

Forward
5'-AGGCCCTCACAGTGTCTTCT-3' (SEQ ID NO: 21)

Reverse
5'-ACATCTTGAATCCCCCAAAG-3' (SEQ ID NO: 22)

[ALDH1A3]

Forward
5'-TGGATCAACTGCTACAACGC-3' (SEQ ID NO: 23)

Reverse
5'-CACTTCTGTGTATTCGGCCA-3' (SEQ ID NO: 24)

[IL6]

Forward
5'-AGACAGCCACTCACCTCTTCAGA-3' (SEQ ID NO: 25)

Reverse
5'-CAGCCATCTTTGGAAGGTTCA-3' (SEQ ID NO: 26)

[36B4]

Forward
5'-CCACGCTGCTGAACATGCT-3' (SEQ ID NO: 27)

Reverse
5'-GATGCTGCCATTGTCGAACA-3' (SEQ ID NO: 28)

<<Immunohistochemical staining>>

The formalin-fixed tissue sample was embedded in paraffin, and the formalin-fixed, paraffin-embedded sample was sliced to prepare a cut piece. For immunohistochemical staining of DPP4, a Histofine kit (NICHIREI CORPORATION) using streptavidin-biotin amplification was used. As primary antibodies, Ki67 (dilution 1:100; MIB1) and DPP4 (dilution 1:100; AF1180-SP) were used. Secondary antibodies were acquired from Agilent Technologies and R&D Systems. The evaluation of immunostaining was carried out by a specialized pathologist.

<<Chromatin immunoprecipitation>>

Chromatin immunoprecipitation (ChIP) was carried out according to the method disclosed in "Namekawa, T, et al. ALDH1A1 in patient-derived bladder cancer spheroids activates retinoic acid signaling leading to TUBB3 overexpression and tumor progression. Int. J. Cancer 146, 1099-1113 (2019)."
The sequences of the primers used for the chromatin immunoprecipitation PCR are as follows.

[Peak]

Forward

5'-CTAATGTGTTGGCCAGCTGCTACC-3' (SEQ ID NO: 29)

Reverse

5'-GTCCGATGGGGCATTACCACATGA-3' (SEQ ID NO: 30)

[Reference]

Forward

5'-CCTCTTCAAGGGGACTAACTACAA-3' (SEQ ID NO: 31)

Reverse

5'-CTATCATTGCAAGATTGCTCTCTC-3' (SEQ ID NO: 32)

<<Luciferase assay>>

A retinoic acid responsive element (RARE) determined by JASPAR (Wasserman W. W. & Sandelin A. Applied bioinformatics for the identification of regulatory elements. Nat. Rev. Genet. 5, 276-287 (2004)) and a mutant oligonucleotide thereof, or RARE direct repeat (DR5) (Bulens, F. et al., Retinoic acid induction of human tissue-type plasminogen activator gene expression via a direct repeat element (DR5) located at -7 kilobases. J. Biol. Chem. 270, 7167-7175 (1995)) were generated by annealing corresponding oligonucleotides (see the sequences of the primers below), and were inserted into a pGL3 promoter (Promega K.K.). A renilla luciferase signal was used as an internal standard, and the luciferase activity was analyzed by Dual-Luciferase Reporter Assay System (Promega K.K.).

The sequences of the primers used for the luciferase assay are as follows.

RARE_WT_U
5'-CTCTAGAAAGACTGGTGACACAGAGGTCAAGGTGGGGGAGGCATGA-3' (SEQ ID NO: 33)

RARE_WT_D
5'-CATGGAGATCTTTCTGACCACTGTGTCTCCAGTTCCACCCCCTCCGTACTGCGC-3' (SEQ ID NO: 34)

RARE_Mut_U
5'-CTCTAGAAAGACCAACATCACAGCAACATAGGTGCAACATGCATGA-3' (SEQ ID NO: 35)

RARE_Mut_D
5'-CGCGTCATGCATGTTGCACCTATGTTGCTGTGATGTTGGTCTTTCTAGAGGTAC-3' (SEQ ID NO: 36)

<<Animal testing>>

All of the animal tests performed was approved by Animal Management and Use Committee of Saitama Medical University, and was carried out according to the guidelines and regulations for the management and use of test animals, set by Saitama Medical University. Male nude mice (BALB/c-nu/nu, 6 weeks old) were acquired from CLAIR Japan, Inc. The ACHN cells and the ACHN-R cells were each subjected to trypsinization, followed by washing with PBS. Then, $5 \times 10^6$ cells of the ACHN cells or the ACHN-R cells were dispersed in 150 μL of Matrigel (BD Biosciences), and were subcutaneously inoculated in each mouse. The size of the tumor was measured every day. The tumor volume (V) was determined by measuring the length (L) and the width (W), and inserting the measured values in the following equation.

$$V = (L \times W^2) \times 0.52$$

When the volume of the engrafted tumor reached 180 mm$^3$, SUN (20 mg/kg) was orally administered to the mice inoculated with the ACHN cells, and SUN (20 mg/kg) alone or two drugs, SUN (20 mg/kg) and DPP4 inhibitor sitagliptin (SITA) (30 mg/kg), were orally administered to the randomly selected mice inoculated with the ACHN-R cells. As a solvent for each drug, a solution including carboxy methyl cellulose sodium (0.5%wt/vol), NaCl (1.8%wt/vol), Tween 80 (0.4%wt/vol), and benzyl alcohol (0.9%wt/vol) was used according to the literature (Stany, M. P. et al. Identification of novel therapeutic targets in microdissected clear cell ovarian cancers. PLoS One 6, e21121 (2011)). The drug was administered in a cycle consisting of 2 consecutive days of administration, followed by 1 day of washout.

<<Statistical analysis>>

For the clinical data, the long rank test was used for the Kaplan-Meier method. As the post-hoc test of multiple comparison, the Holm-Bonferroni method was used. For the partial response rate and characteristics of the patients, the Fisher's exact test was used. For the maximum tumor shrinkage, the Mann-Whitney U test was used. In the cell proliferation assay or quantitative reverse transcription PCR, the two-tailed student's t-test was used for comparison between 2 groups, and the two-way ANOVA was used for multiple comparison. For statistical calculations, JMP 9.0.0 (SAS Institute Japan, Ltd.) was used.

<Results>

<<Correlation between expression level of DPP4 and expression level of cancer stem-like cell related gene in RCC stem-like cells>>

In order to investigate the correlation between the expression of DPP4 and the expression of the cancer stem-like cell (CSC) related gene in the RCC stem-like cells, PDC was produced from 15 cases of clear cells RCC by 3D spheroid culture employing a technique of culturing regular tissue stem cells. The expression level of DPP4 was evaluated by quantitative reverse transcription PCR. As the internal standard, 36B4 was used. The Spearman' s rank correlation coefficient(R) and statistical significance (P) were calculated using JMP software ver. 9.0.0.0 of SAS Institute Japan, Ltd.

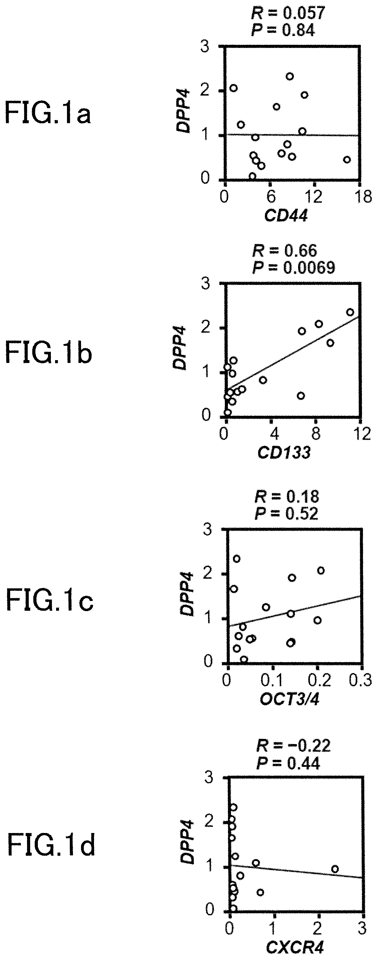

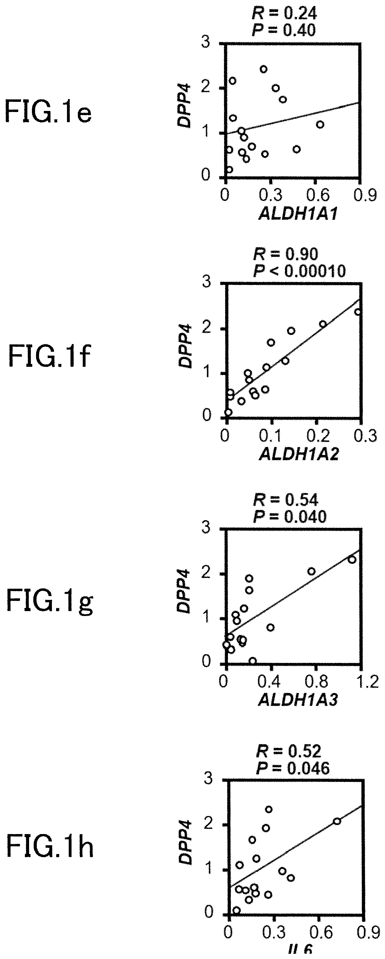

The results are presented in Figs. 1a to 1h.

As depicted in Figs. 1a to 1h, the mRNA expression level of DPP4 was significantly correlated with the mRNA expression levels of CD133, aldehyde dehydrogenase 1 (ALDH1) A2, ALDH1A3, and IL6, respectively.

Next, the hematoxylin-eosin (HE) staining and DPP4 immunohistochemical staining were performed to study the morphologies of the original tumors of RCC-A and RCC-B, which were typical PDC.

The results are presented in Table 2a.

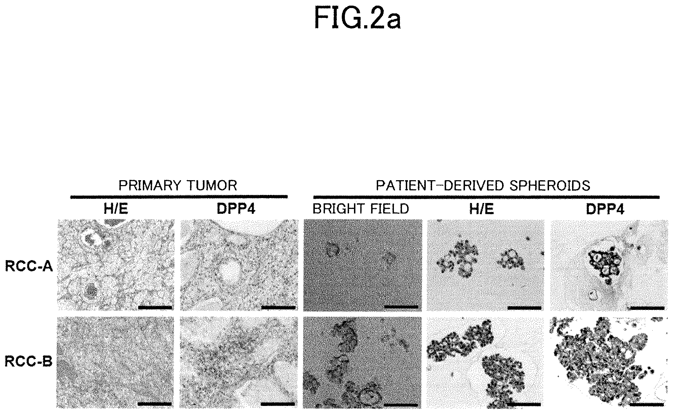

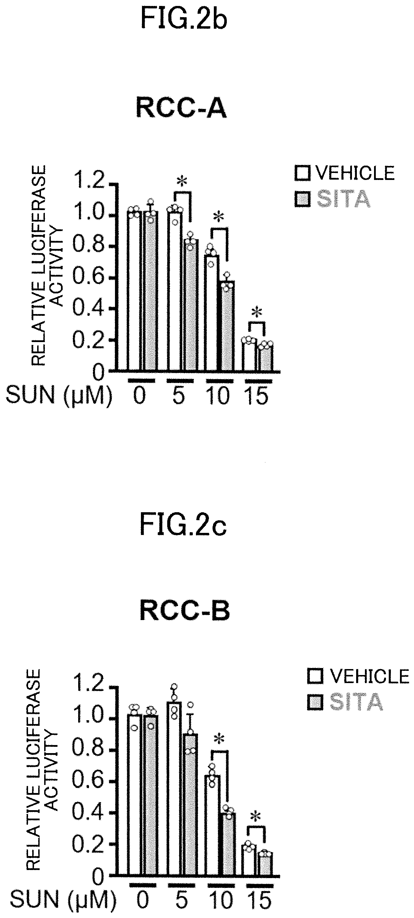

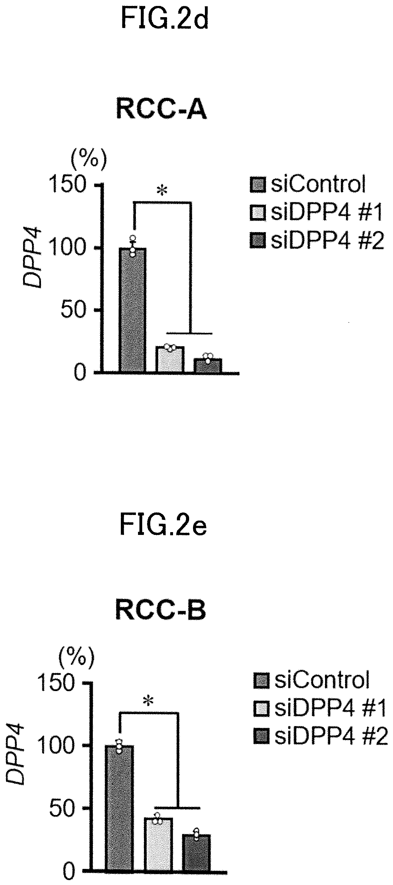

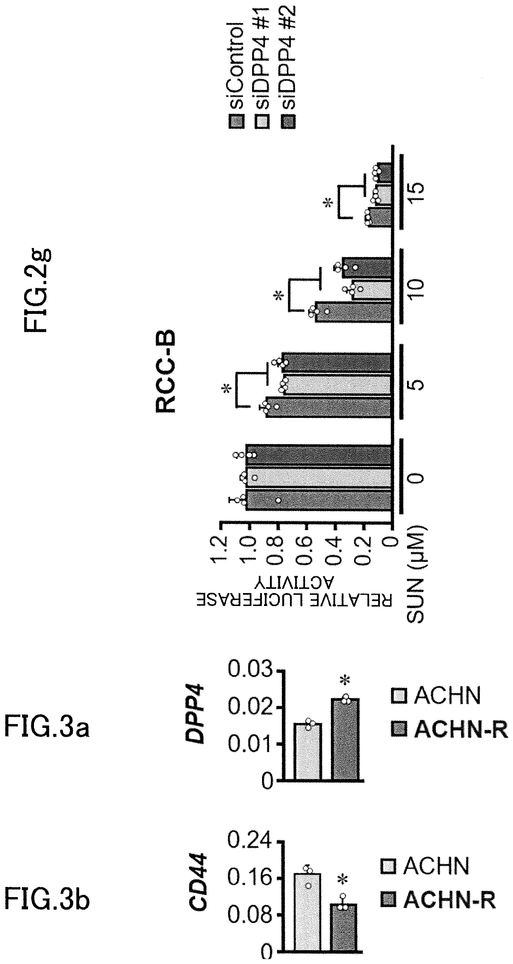

As depicted in Fig. 2a, PDC maintained the morphologies of tumor cells similar to those of the original tumors cell, and the DPP4 staining was also positive. The RCC-A and RCC-B were also provided for further experiments below.

<<Enhancement of tumor proliferation effect of sunitinib on RCC cells by inhibition of DPP4 in RCC patient-derived spheroid culture>>

In order to evaluate whether DPP4 was related to properties of cancer stem-like cells, and contributed to survival of cancer cells, an experiment was carried out using a multi-target receptor tyrosine kinase inhibitor sunitinib (SUN), which was known as a medicine for RCC. In order to investigate how inhibition of DPP4 affected therapeutic effects of SUN, DPP4 inhibitor SITA, which had been already known as a therapeutic drug for Type 2 diabetes mellitus (T2DM), or siRNA (siDPP4 #1 and #2) whose target was DPP4 was used in combination with SUN to evaluate the effect on proliferation of spheroids.

Figs. 2b to 2c depict the results of treating the RCC-A spheroids and the RCC-B spheroids with SITA (100 $\mu$M) and SUN. The data was presented as the mean value $\pm$ SD of the relative luciferase activity as analyzed by an ATP-based luciferase assay. A two-tailed student's t-test was carried out to compare between a solvent and SITA with setting a level of significance as P < 0.05. Note that, in Figs. 2b to 2c, each pair of bars along the horizontal axis of each bar graph represents the result of the cells treated with the vehicle and the result of the cells treated with SITA in the order from the left side to the right side. In Figs. 2b to 2c, moreover, "*" represents P < 0.05.

The production of the spheroid ATP in the RCC-A cells or RCC-B cells treated with SUN alone or a combination of SUN and SITA on the third day was measured with the luciferase activity using the CEllTiter-Glo. As depicted in Figs. 2b to 2c, the proliferation inhibition effect owing to SUN was enhanced when SITA was used in combination with SUN.

Figs. 2d (RCC-A) to 2e(RCC-B) depict the results of the DPP4 expression levels determined in the RCC-A spheroids or the RCC-B spheroids treated with control siRNA (siControl) or DPP4-specific siRNA (siDPP4 #1 or #2). The data was presented as percentage of the mean $\pm$ SD of the relative DPP4 level in each group of the spheroids treated with siRNA. A two-tailed student's t-test was carried out to compare siControl and DPP4-specific siRNA (siDPP4) under each condition with setting a level of significance as P < 0.05. Note that, in Figs. 2d to 2e, each set of bars along the horizontal axis of each bar graph represents the result of the cells treated with siControl, the result of the cells treated with siDPP4 #1, and the result of the cells treated with siDPP4 #2 in the order from the left side to the right side. In Figs. 2d to 2e, moreover, "*" represents P < 0.05.

As depicted in Figs. 2d to 2e, it was confirmed that expression of DPP4 was suppressed by siDPP4.

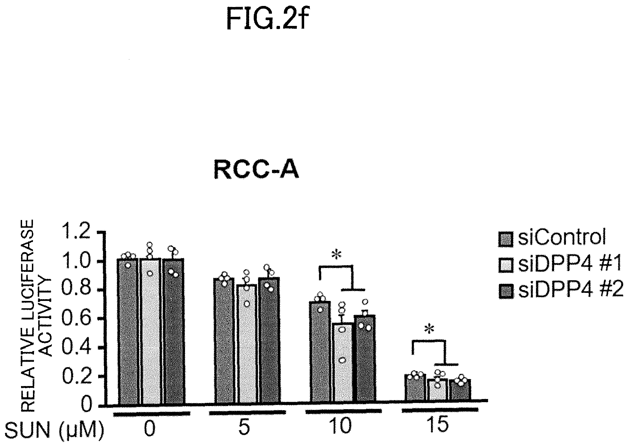

Figs. 2f (RCC-A) to 2g (RCC-B) depict the results of the effect of the DPP4-specific siRNA to the cell survival rate of the RRC-A or RCC-B spheroids treated with SUN. The data was presented as the mean value $\pm$SD of the relative luciferase activity in each spheroid culture analyzed by an ATP-based luciferase assay. A two-tailed student's t-test was carried out to compare siControl and siDPP4 under each condition with setting a level of significance as P < 0.05. Note that, in Figs. 2f to 2g, each set of 3 bars along the horizontal axis of each bar graph represents the result treated with siControl, the result treated with siDPP4 #1, and the result treated with siDPP4 #2 in the order from the left side to the right side. In Figs. 2f to 2g, moreover, "*" represents P < 0.05.

As depicted in Figs. 2f to 2g, if the expression of DPP4 was suppressed by siDPP4, the proliferation inhibition effect owing to SUN was also enhanced.

Next, ACHN-R cells and 769-P-R cells, which were both SUN resistant RCC cell lines, were experimentally produced using ACHN cells and 769-P cells, which were both RCC cell lines, respectively. The expression level of the mRNA of the DPP4 and the expression level of the mRNA of the CSC-related gene were compared between the ACHN-R cells and the parent cell line thereof, or between the 769-P-R cells and the parent cell line thereof.

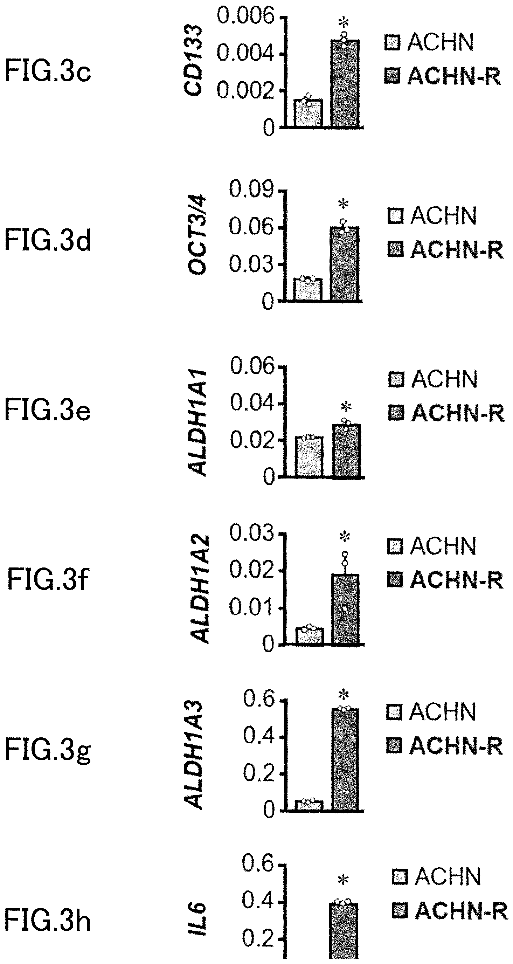

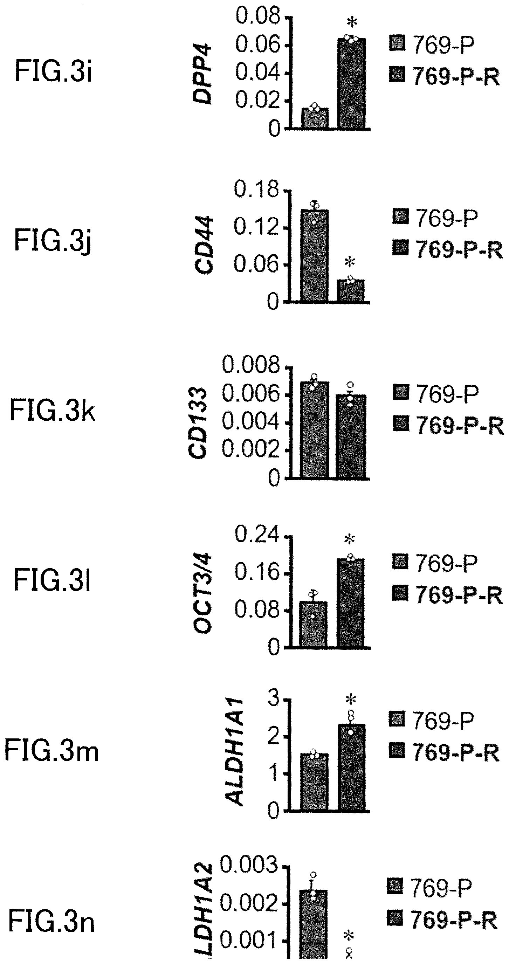

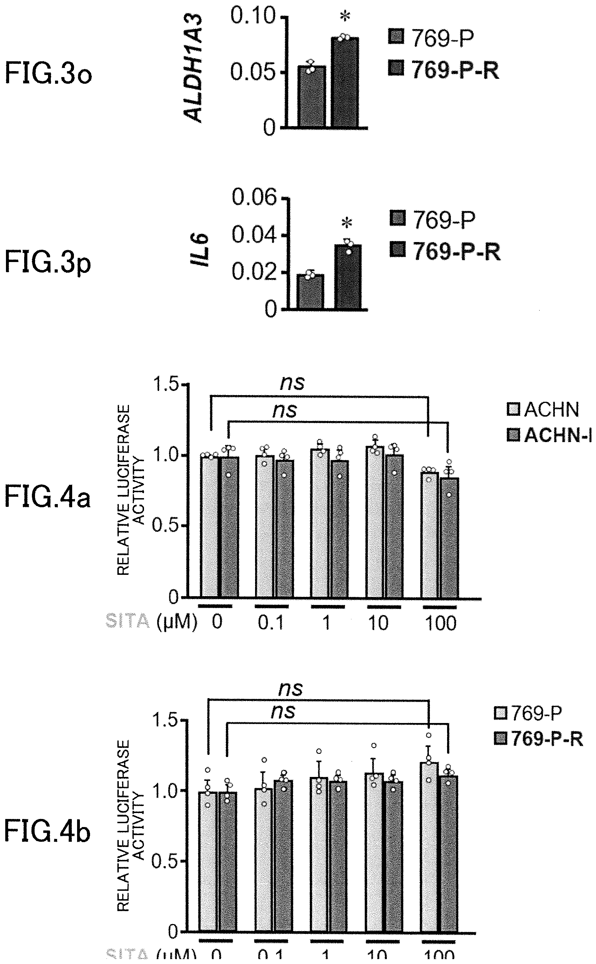

Figs. 3a to 3h depict the results evaluating the expression levels of DPP4 and the CSC-related genes in the 3D culture of the ACHN cells and the 3D culture of the ACHN-R cells by qRT-PCR. Figs. 3i to 3p depict the results evaluating the expression levels of the DPP4 and the CSC-related genes in the 3D culture of the 769-P cells and the 3D culture of the 769-P-R cells by qRT-PCR. The data was represented as the mean $\pm$ SD, and n=3. A two-tailed student's t-test was carried out with setting a level of significant as P < 0.05. Note that, in Figs. 3a to 3p, the bars along the horizontal axis of each graph represent the result of the parent cell line and the result of the resistant cell line in the order from the left side to the right side. In Figs. 3a to 3j and 3l to 3p, moreover, "*" represents P < 0.05.

As depicted in Figs. 3a to 3p, the expression levels of the mRNA of all DPP4, OCT3/4, ALDH1A1, ALDH1A3, and IL6 were increased in the resistant cell lines.

Moreover, SITA or siDPP4 was used in combination with SUN in the ACHN-R cells or 769-P-R cells to evaluate an effect to the proliferation of spheroids.

First, a dose-response effect of sitagliptin (SITA) to 3D spheroid proliferation of ACHN-R, 769-P-R, or a parent cell line thereof was investigated with administration of SITA alone.

Fig. 4a depicts the results of ACHN-R and the parent cell line thereof. Fig. 4b depicts the results of 769-P-R and the parent cell line thereof. The data was presented as the mean ± SD, and n=4. A two-tailed student's t-test was carried out with setting a level of significance as P < 0.05. Note that, in Figs. 4a to 4b, each pair of bars for each item along the horizontal axis of each bar graph represents the result of the parent cell line, and the result of the resistant cell line in the order from the left side to the right side. In Figs. 4a to 4b, moreover, "ns" represents "no significant difference."

As presented in Figs. 4a to 4b, administration of SITA alone did not affect the spheroid proliferation of the ACHN cells, ACHN-R cells, 769-P cells, nor 769-P-R cells.

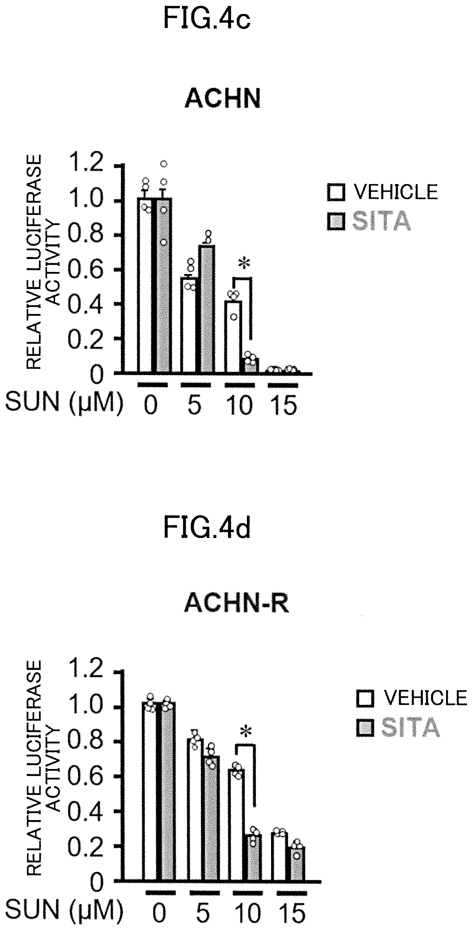

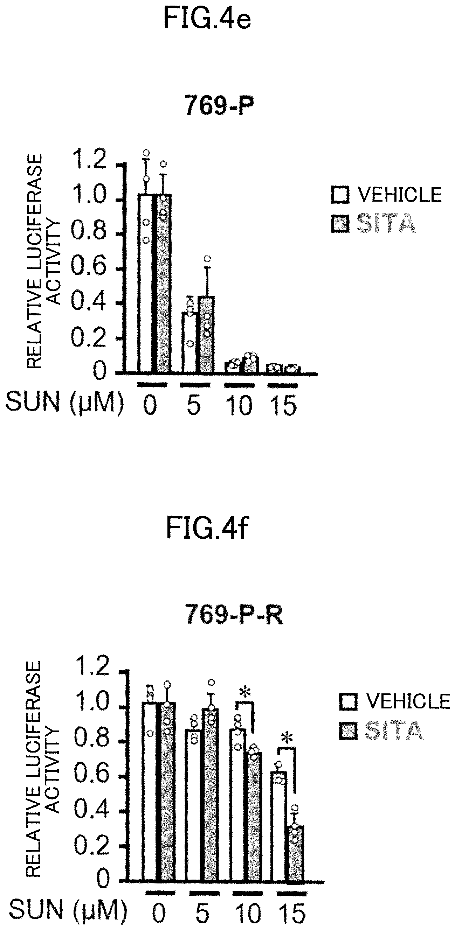

Figs. 4c to 4f are diagrams depicting the results of studying the effect of the combination of SITA and SUN in the 3D culture of the ACHN cells (Fig. 4c), ACHN-R cells (Fig. 4d), 769-P cells (Fig. 4e), or 769-P-R cells (Fig. 4f). The concentration of SITA used was 100 μM. Note that, in Figs. 4c to 4f, each pair of bars for each item along the horizontal axis of each bar graph represents the result treated with the vehicle and the result treated with SITA in the order from the left side to the right side. In Figs. 4c, 4d, and 4f, moreover, "*" represents P < 0.05.

As depicted in Figs. 4c to 4f, the administration of the combination of SUN and SITA suppressed the spheroid proliferation of the ACHN-R cells and the spheroid proliferation of the 769-P-R cells.

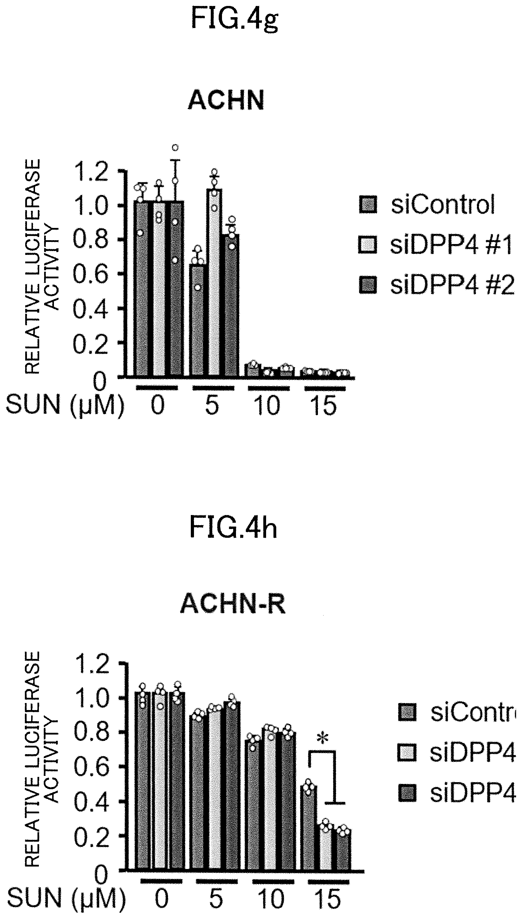

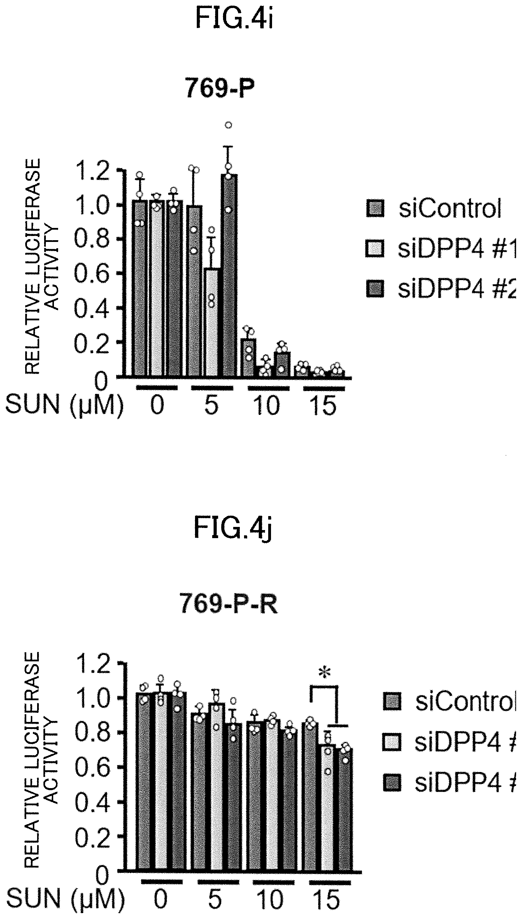

Figs. 4g to 4j are diagrams depicting the results studying how the DPP4 knockdown by siRNA affects the therapeutic effects of SUN in the 3D culture of the ACHN cells (Fig. 4g), ACHN-R cells (Fig. 4h), 769-P cells (Fig. 4i), or 769-P-R cells (Fig. 4j). The data was represented as the means value ±SD of the relative luciferase activity of each group of the spheroids, and n=3. Note that, in Figs. 4g to 4j, each set of three bars for each item along the horizontal axis of each bar graph represents the result of the cells treated with siControl, the result of the cells treated with siDPP4 #1, and the result of the cells treated with siDPP4 #2 in the order from the left side to the right side. In Figs. 4h to 4j, moreover, "*" represents P < 0.05.

As depicted in Figs. 4g to 4j, it was confirmed that suppression of expression of DPP4 by siDPP4 enhanced the proliferation inhibition effect of SUN both in the ACHN-R cells and the 769-P-R cells to enhance the therapeutic effects of SUN.

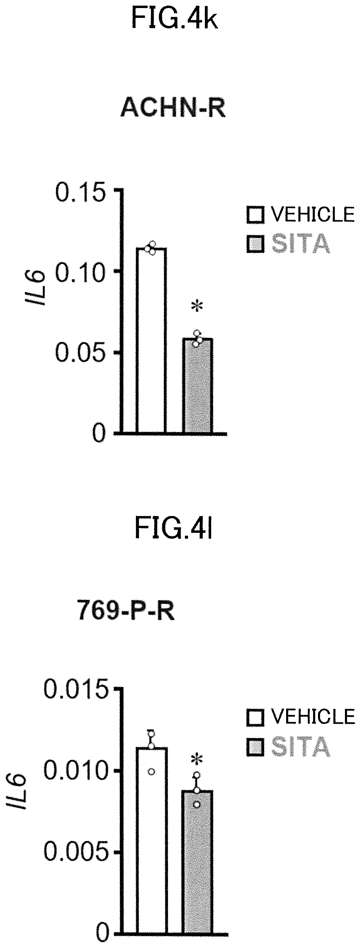

Figs. 4k to 4l depict the results studying how SITA treatment affects the IL6 mRNA level in the 3D cultures of the ACHN-R cells (Fig. 4k) and 769-P-R cells (Fig. 4l), which are both SUN resistant cells. The data was presented as the mean ±SD using 36B4 as the internal standard, and n=3. A two-tailed student's t-test was carried out with setting a level of significance as P < 0.05. Note that, in Figs. 4k to 4l, a pair of bars for each item along the horizontal axis of each bar graph represents the result of the cells treated with the vehicle and the result of the cells treated with SITA in the order from the left side to the right side. In Figs. 4k to 4l, moreover, "*" represents P < 0.05.

As depicted in Figs. 4k to 4l, the DPP4 inhibitor reduced the expression of mRNA of IL6.

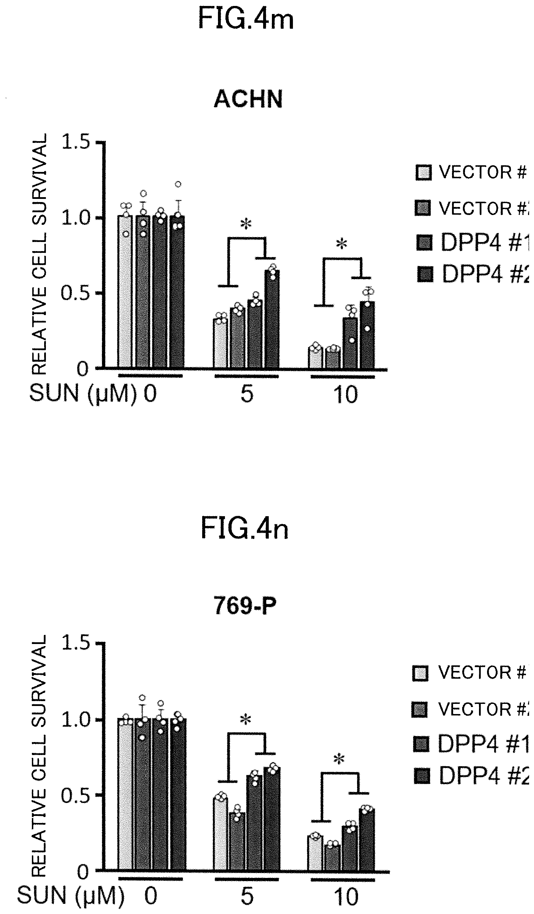

Figs. 4m to 4n are diagrams depicting the results studying how the DPP4 overexpression affects the cell survival rate of the RCC spheroids treated with SUN. ACHN cells (Fig. 4m) and 769-P cells (Fig. 4n), each stably expressing a control vector (vector #1 or #2) or DPP4 (DPP4 #1 or #2) were treated with SUN, and were analyzed by an ATP-based luciferase assay. The data was presented as the mean value ±SD of the relative luciferase activity of each group of spheroids, and n=4. A two-tailed student's t-test was carried out with setting a level of significance as P < 0.05. Note that, in Figs. 4m to 4n, each set of 4 bars along the horizontal axis of each bar graph represents the result of the cells processed with the vector #1, the result of the cells processed with the vector #2, the result of the cells processed with DPP4 #1, and the result of the cells processed with DPP4 #2 in the order from the left side to the right side. In Figs. 4m to 4n, moreover, "*" represents P < 0.05.

As depicted in Figs. 4m to 4n, it was confirmed that the cell survival of the ACHN cells and 769-P cells where DPP4 was stably overexpressed was improved by the administration of SUN, and the DPP4 overexpression recovered the cell survival rates of RCC spheroids by SUN.

<<Control of DPP4 expression in SUN resistant RCC cells by retinoic acid signal>>

Since the expression level of ALDH1 and the expression level of DPP4 were correlated, a hypothesis was made that the function of ALDH1 might be related to expression of DPP4. ALDH1 is an enzyme related to metabolization of retinol. ALDH1 converts retinol to retinoic acid (RA) to act as a ligand for an RA receptor, such as a retinoic acid receptor α (RARα) etc.

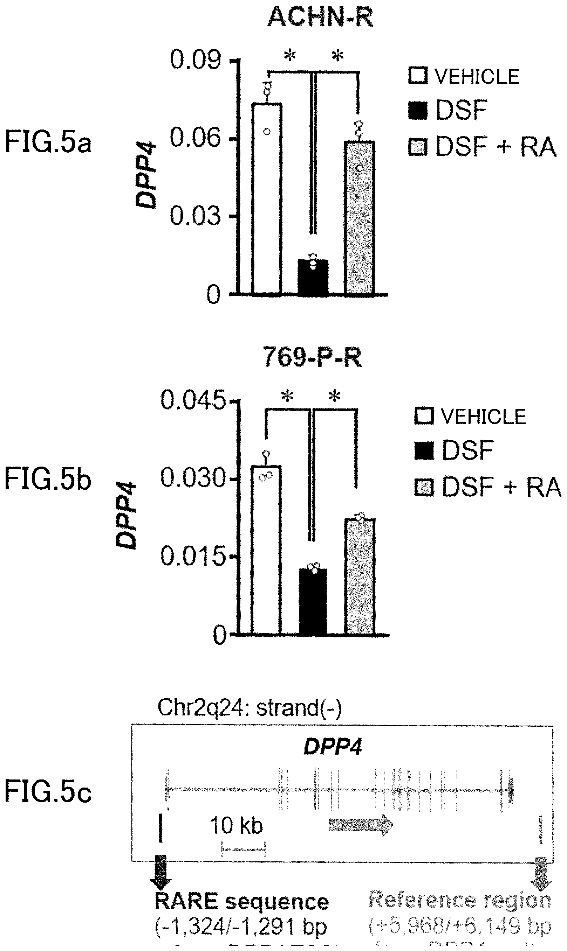

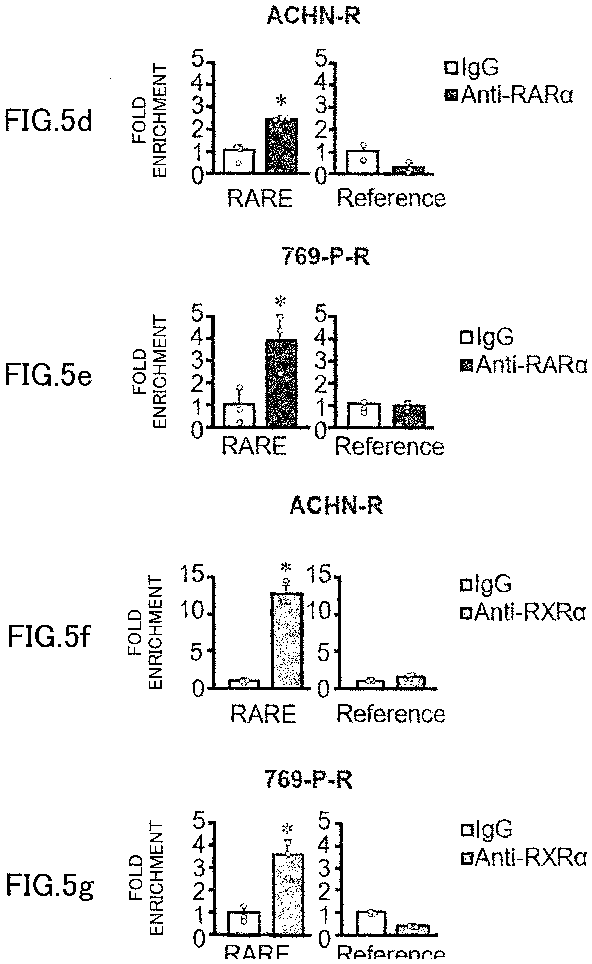

Figs. 5a to 5b are diagrams depicting the results studying how addition of RA to sunitinib (SUN) resistant RCC cells (Fig. 5a: ACHN-R cells, Fig. 5b: 769-P-R cells) affects suppression of expression of DPP4 by disulfiram (DSF), which is one of the FDA-approved ALDH inhibitors used for treatment of alcoholism. The ACHN-R cells were treated with a vehicle alone, or 15 $\mu$M of DSF, or 15 $\mu$M of DSF and 1 $\mu$M of RA. The 769-P-R cells were treated with a vehicle alone, or 25 $\mu$M of DSF, or 25 $\mu$M of DSF and 1 $\mu$M of RA. The data was presented as the mean $\pm$ SD, and n=3. A two-tailed student's t-test was carried out with setting a level of significance as P < 0.05. Note that, in Figs. 5a to 5b, the bars along the horizontal axis of each bar graph represent the result of the cells treated with the vehicle, the result of the cells treated with DSF, and the result of the cells treated with DSF and RA in the order from the left side to the right side. In Figs. 5a to 5b, moreover, "*" presents P < 0.05.

As depicted in Figs. 5a to 5b, it was found that, as disulfiram (DSF) was added, expression of DPP4 was suppressed in both the ACHN-R cells and the 769-P-R cells, but this effect was reversed by addition of RA. This result indicates that the RA signal controls DPP4 expression in the RCC cells.

Next, in order to determine the RAR$\alpha$ bonding site in the DPP4 gene promoter region, the promoter region of DPP4 searched from the hg19 human genome dataset was searched with the matrix profile of RARE using an open-access transcription factor binding profile database JASPAR (http://jaspar.genereg.net/)(Wasserman W. W. & Sandelin A. Applied bioinformatics for the identification of regulatory elements. Nat. Rev. Genet. 5, 276-287 (2004)).

One RARE candidate was determined in the DPP4 promoter region that was from the position of the transcription start site (TSS) to -1619 bp, and to -1647 bp, where the threshold of the relative profile score of the JASPAR algorithm exceeded 85% (Fig. 5c).

Figs. 5d to 5e depict the results confirming the recruit of the retinoic acid receptor $\alpha$ (RAR$\alpha$) to RARE in the DPP4 promoter of the SUN resistant RCC cells. Figs. 5f to 5g depict the results confirming the recruit of the retinoid X receptor $\alpha$ (RXR$\alpha$) to RARE in the DPP4 promoter of the SUN resistant RCC cells. The chromatin immunoprecipitation of the ACHN-R cells and 769-P-R cells was carried out using the anti-RAR$\alpha$ antibody (Figs. 5d to 5e) or anti-RXR$\alpha$ antibody (Figs. 5f to 5g) and the control IgG, and the quantification was carried out by PCR. The data was presented as the mean $\pm$ SD, and n = 3. A two-tailed student's t-test was carried out with setting a level of significance as P < 0.05. Note that, in Figs. 5d to 5g, the bar graph on the left side depicts the results of RARE, and the bar graph on the right side depicts the results of the reference region. In Figs. 5d to 5e, the bars along the horizontal axis of each bar graph depict the result when IgG was used, and the result when the RAR$\alpha$ antibody was used in the order from the left side to the right side. In Figs. 5f to 5g, the bars along the horizontal axis of each bar graph depict the result when IgG was used, and the result when the RXR$\alpha$ antibody was used in the order from the left side to the right side. In Figs. 5d to 5g, moreover, "*" represents P < 0.05.

As depicted in Figs. 5d to 5g, by carrying out the ChIP-qPCR assay the RAR$\alpha$ antibody, the RXR$\alpha$ antibody, and IgG, it became clear that both RAR$\alpha$ and RXR$\alpha$ were recruited to RARE in both the ACHN-R cells and the 769-P-R cells.

Next, a luciferase reporter assay was carried out to investigate whether RA modified the DPP4 promoter activity. A luciferase reporter including wild-type (WT) RARE, and a luciferase reporter including mutated (Mut) RARE were used. Moreover, as known RARE, RARE having a direct repeat 5 (DR5) (Bulens, F. et al. Retinoic acid induction of human tissue-type plasminogen activator gene expression via a direct repeat element (DR5) located at -7 kilobases. J. Biol. Chem. 270, 7167-7175 (1995)) was inserted into a luciferase reporter, and the resulting luciferase reporter was used as a positive control.

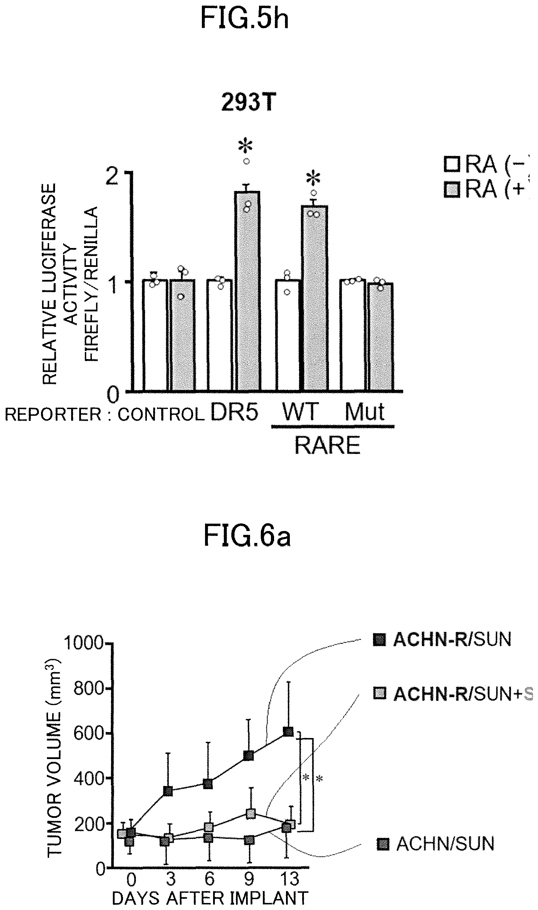

The results are presented in Fig. 5h. The data was presented as the mean $\pm$ SD, and n = 3. A two-tailed student's t-test was carried out with setting a level of significance as P < 0.05. Note that, in Fig. 5h, a pair of bars along the horizontal axis of each bar graph depicts the result without the RA treatment and the result with the RA treatment in the order from the light side to the right side. In Fig. 5h, moreover, "*" represents P < 0.05.

As depicted in Fig. 5h, a significance of the transcriptional activity of the wild-type (WR) RARE in the 293T cells was increased by responding to the RA treatment, conversely, the activation of the mutated (Mut) RARE by the RA treatment was not observed. The results suggested that the RA/RAR/RXR signals controlled transcription of DPP4 via the functional RARE.

<<Inhibition of proliferation of SUN resistant RCC xenograft tumor by DPP4 inhibitor SITA>>

An effect of SITA used in combination on proliferation of SUN resistant RCC tumors was examined using a xenograft tumor model.

At the point when the volume of the ACHN or ACHN-R xenograft nude mouse tumor reached 180 mm$^3$, oral administration of SUN alone, or a combination of SUN and SITA was started.

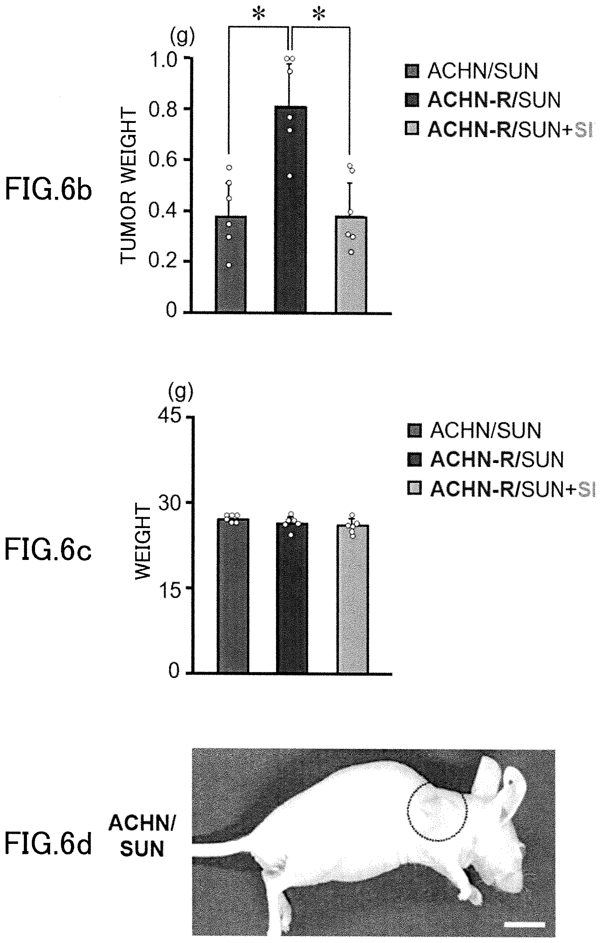

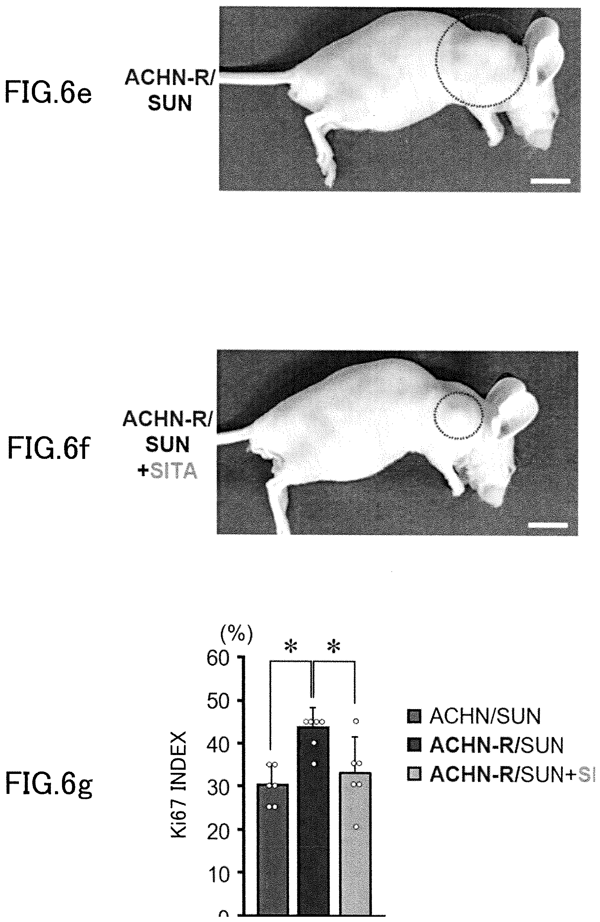

The proliferation curves of the ACHN xenograft tumor treated with sunitinib (SUN) (ACHN/SUN group, n = 6), the ACHN-R xenograft tumor treated with SUN (ACHN-R/SUN group, n = 6), and the ACHN-R xenograft tumor treated with SUN and SITA (ACHN-R/SUN+SITA group, n = 6) are depicted in Fig. 6a, the tumor weights of the mice of all the groups on the 13th day (endpoint) are depicted in Fig. 6b, and the weights of the mice of all the groups on the 13th day are depicted in Fig. 6c. The data was presented as the mean $\pm$ SD, and n = 6. A two-tailed student's t-test was carried out with setting a level of significance as P < 0.05. The tumor volume was estimated by measuring the size of the tumor with Vernier calipers. Note that, in Figs. 6b to 6c, the bars along the horizontal axis of each bar graph depict the result of the ACHN/SUN group, the result of the ACHN-R/SUN group, and the result of the ACHN-R/SUN+SITA group in the order from the left side to the right side. In Figs. 6a and 6b, moreover, "*" represents P < 0.05.

Moreover, typical photographs of the xenograft nude mice models of all groups on the 13th day are depicted in Figs. 6d to 6f. The scale bar indicates 10 mm.

As depicted in Figs. 6a to 6f, the ACHN-R xenograft tumor demonstrated stronger resistance to SUN compared to the ACHN xenograft tumor, but the proliferation of the tumor was suppressed to the similar degree to the ACHN xenograft tumor by using SUN and SITA in combination.

Ki67 immunohistochemical staining of the excised tumor of each group was carried out to determine a ratio of Ki67 positive cells (Ki67 index). The result is depicted in Fig. 6g. The scale bar is 50 um. The data was presented as the mean $\pm$ SD, and n = 6. A two-tailed student's t-test was carried out with setting a level of significance as P < 0.05. Note that, in Fig. 6g, the bars along the horizontal axis of the bar graph depict the result of the ACHN/SUN group, the result of the ACHN-R/SUN group, and the result of the ACHN-R/SUN+SITA group from the left side to the right side. In Fig. 6g, moreover, "*" represents P < 0.05.

As presented in Fig. 6g, use of SITA and SUN in combination reduced the Ki67 index, which was the cell proliferation marker, in the ACHN-R xenograft tumor.

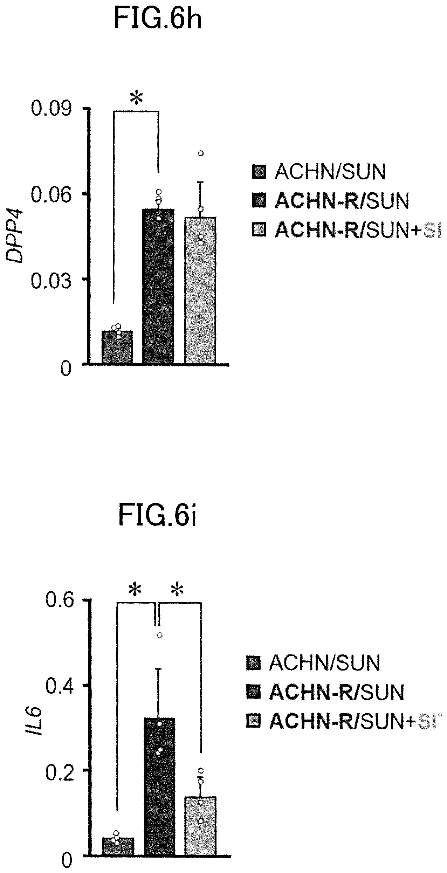

Fig. 6h (DPP4) and Fig. 6i (IL6) depict the results studying the mRNA levels of DPP4 and IL6 in each group. The expression level was evaluated by qRT-PCR. The data was presented as the mean $\pm$ SD, and n = 6. A two-tailed student's t-test was carried out with setting a level of significance as P < 0.05. Note that, in Figs. 6h to 6i, a set of bars along the horizontal axis of each bar graph depicts the result of the ACHN/SUN group, the result of the ACHN-R/SUN group, and the result of the ACHN-R/SUN+SITA group in the order from the left side to the right side. In Figs. 6h to 6i, moreover, "*" represents P < 0.05.

As presented in Figs. 6h to 6i, DPP4 expression and IL6 expression increased in the ACHN-R tumor compared to the ACHN tumors, and the expression of IL6 decreased in the ACHN-R xenograft tumor to which SITA was used in combination.

It was confirmed from the results described above that the sitagliptin (SITA) contributed to overturn the sunitinib resistance of the RCC xenograft tumor.

<<Contribution of DPP4 inhibitor (DPP4i) on desirable prognosis of RCC patient who received TKI treatment>>

To investigate how the data presented by the in vitro tests and In vivo tests above relates to the prognosis in the clinical cases or drug efficacy, a retrospective clinical study was carried out on the 73 RCC cases of the patients who received the TKI (sunitinib, sorafenib, axitinib, or pazopanib) treatment at Saitama Medical Center, Saitama Medical University. Within the 73 cases, the cases where T2DM was not present, and DPP4i (sitagliptin, linagliptin, alogliptin, teneligliptin, anagliptin, or vildagliptin) was not administered were 47 cases (T2DM-/DPP4i-), the cases where T2DM was present and DPP4i was not administered were 12 cases (T2DM+/DPP41-), and the cases where T2DM was present and DPP4i was administered were 14 cases (T2DM+/DPP4i+).

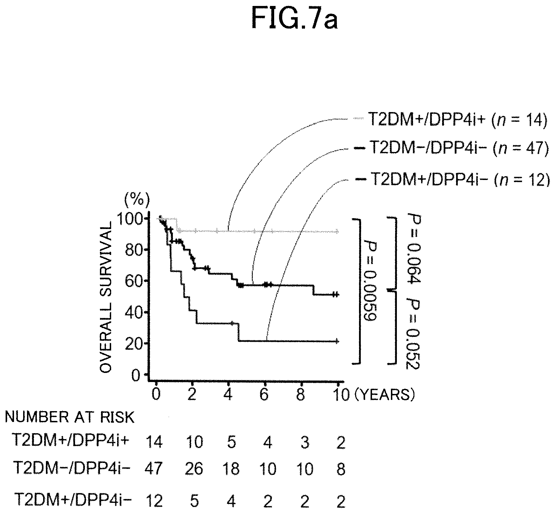

Fig. 7a depicts the results of the Kaplan-Meier analysis performed on the 10-year overall survival of the above-mentioned 3 groups to study whether the administration of DPP4i contributed prognosis. The P value was evaluated by a long-rank test. As the post-hoc test of multiple comparison, the Holm-Bonferroni method was used.

As depicted in Fig. 7a, the administration of DPP4i was a favorable prognosis factor for the T2DM patients. It was confirmed that the DPP4 inhibitor (DPP4i) contributed to the prognosis of the RCC patients who received the TKI treatment and to the maximum tumor shrinkage.

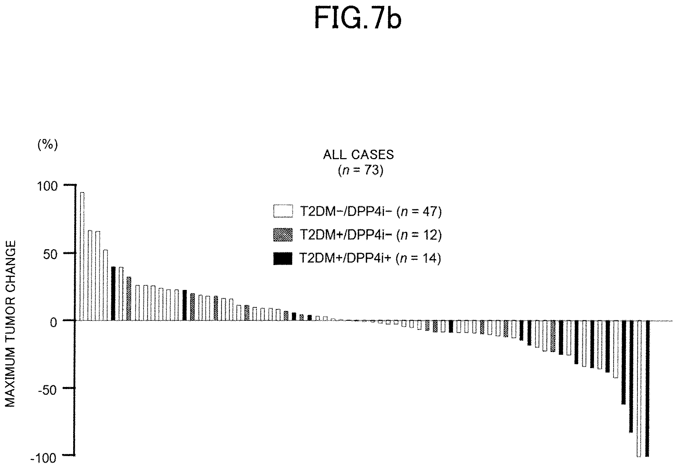

Next, Fig. 7b depicts the result of the waterfall plot analysis of the maximum tumor change of the 73 RCC patients who received the stratified TKI treatment depending on the existence or nonexistence of T2DM and administration or no administration of the DPP4i. The partial response rates of all the groups were compared by the Fisher's exact test. The partial response rate with the TKI treatment was increased by the administration of DPP4i (see Table 1 below).

[Table 1]

| 2×3 contingency table for the partial response rates of 73 cases of the RCC patients received TKI treatment | | | | |
|---|---|---|---|---|
| Tumor response[a] | T2DM-/DPP4i-(n = 47) | T2DM+/DPP4i-(n = 12) | T2DM+/DPP4i+ (n = 14) | P-value[b] |
| PR- <br> PR+ | 43 (91.5) <br> 4 (8.5) | 12 (100) <br> 0 (0) | 8 (57.1) <br> 6 (42.9) | 0.0045 |

[a]: evaluation based on RECIST version 1.1
[b]: analysis according to the Fisher's exact test

PR: partial response

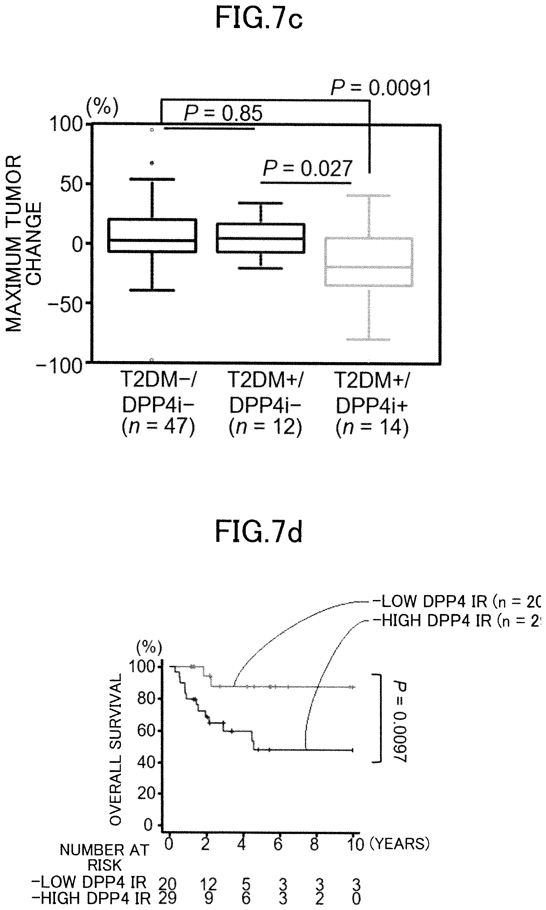

Fig. 7c is a diagram depicting the results of the box plot analysis of the maximum tumor change for the 73 RCC patients who received the stratified TKI treatment depending on the existence or nonexistence of T2DM and administration or no administration of the DPP4i. The P value was evaluated by the Mann-Whitney U test.

As depicted in Fig. 7c, the enhancement of the tumor shrinkage effect by the DPP4i was confirmed.

Moreover, an analysis was carried out on the 49 cases in which immunostaining was possible, within the 73 cases, according to immunohistochemical staining of DPP4 to evaluate the association between the immunostaining of DPP4 and the prognosis or TKI therapeutic effects.

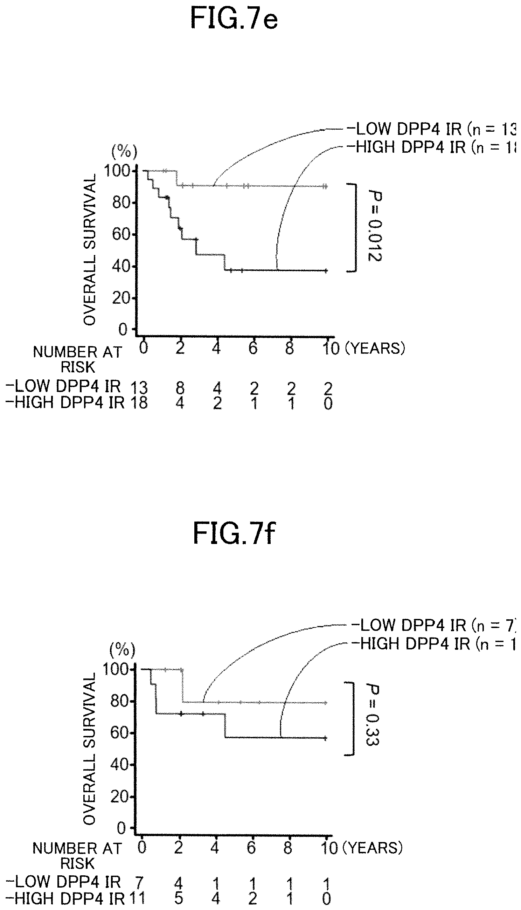

As depicted in Fig. 7d, as Kaplan-Meier analysis of the 10-year overall survival was performed to investigate the relationship between the DPP4 expression and prognosis, the high level of DPP4 expression was an adverse prognostic factor. Even when the analysis was performed only on the 31 non-T2DM cases, the high level of DPP4 expression was an adverse prognostic factor (Fig. 7e). As the analysis was performed only on the 18 T2DM cases, however, the DPP4 expression was not a significant prognosis factor (Fig. 7f). Note that, the P value was evaluated by the long-rank test.

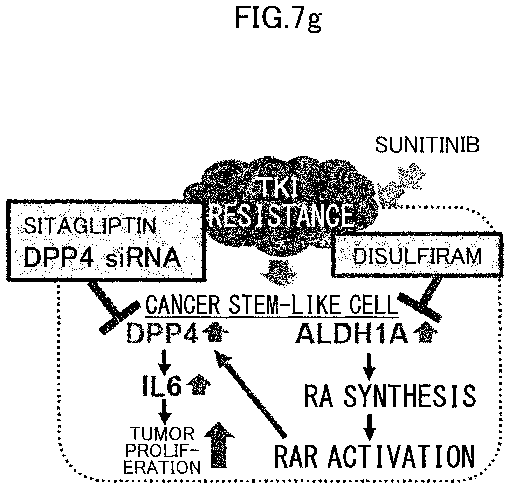

Within the 49 cases, the partial response rate was analyzed on the maximum tumor change of the 20 cases of the RCC patients exhibiting low DPP4 immune response. As a result, a significant difference was not observed (see Table 2 below). On the other hand, a significant difference in the maximum tumor change by the administration of the DPP4 inhibitor was observed in the 29 cases of the RCC patient exhibiting low DPP4 immune response (see Table 3 below). Fig. 7g depicts a schematic diagram of the ALDH1/RA/DPP4 system in the high DPP4 expression RCC stem-like cell according to the present research.

[Table 2]

| 2×3 contingency table for the partial response rates of 20 cases of the RCC patients of low DPP4 immune response | | | | |
|---|---|---|---|---|
| Tumor response[a] | T2DM-/DPP4i-(n = 13) | T2DM+/DPP4i-(n = 3) | T2DM+/DPP4i+ (n = 4) | P-value[b] |
| PR- PR+ | 10 (76.9) 3 (23.1) | 3 (100) 0 (0) | 2 (50.0) 2 (50.0) | 0.46 |

[a]: evaluation based on RECIST version 1.1

[b]:analysis according to the Fisher's exact test

PR: partial response

[Table 3]

| 2×3 contingency table for the partial response rates of 29 cases of the RCC patients of high DPP4 immune response | | | | |
|---|---|---|---|---|
| Tumor response[a] | T2DM-/DPP4i-(n = 18) | T2DM+/DPP4i-(n = 5) | T2DM+/DPP4i+ (n = 6) | P-value[b] |
| PR- PR+ | 17 (94.4) 1 (5.6) | 5 (100) 0 (0) | 3 (50.0) 3 (50.0) | 0.037 |

[a]: evaluation based on RECIST version 1.1

[b]:analysis according to the Fisher's exact test

PR: partial response

For example, the embodiments of the present invention include the following.

<1> A combination drug for treating a renal cancer, the combination drug including:

a combination of
a tyrosine kinase inhibitor, and
a dipeptidyl peptidase-4 inhibitor.

EP 4 260 873 A1

<2> The combination drug according to <1>,
wherein the renal cancer is a renal cancer having resistance to the tyrosine kinase inhibitor.
<3> The combination drug according to <1> or <2>,
wherein the tyrosine kinase inhibitor is at least one selected from the group consisting of sunitinib, sorafenib, axitinib, pazopanib, cabozantinib, and lenvatinib.
<4> The combination drug according to any one of <1> to <3>, wherein the dipeptidyl peptidase-4 inhibitor is at least one selected from the group consisting of sitagliptin, linagliptin, alogliptin, teneligliptin, anagliptin, vildagliptin, saxagliptin, trelagliptin, omarigliptin, gemigliptin, evogliptin, gosogliptin, and a dipeptidyl peptidase-4 expression inhibitor.
<5> The combination drug according to <4>,
wherein the dipeptidyl peptidase-4 expression inhibitor is at least one selected from the group consisting of a double-stranded nucleic acid molecule inhibiting expression of a dipeptidyl peptidase-4 gene, DNA including a base sequence encoding the double-stranded nucleic acid molecule, and a vector including the DNA.
<6> The combination drug according to <5>,
wherein the double-stranded nucleic acid molecule inhibiting expression of the dipeptidyl peptidase-4 gene includes:

(a) a sense strand including a base sequence corresponding to a target sequence composed of a base sequence of SEQ ID NO: 1 or SEQ ID NO: 4; and
(b) an antisense strand that constitutes a double stranded nucleic acid with the sense strand of (a), and includes a base sequence complimentary to the sense strand.

<7> The combination drug according to <5> or <6>,
wherein the double-stranded nucleic acid molecule is double-stranded RNA or a double-stranded RNA-DNA chimera.
<8> The combination drug according to any one of <5> to <7>, wherein the double-stranded nucleic acid molecule is siRNA or chimeric siRNA.
<9> A combination drug according to any one of <5> to <8>, wherein the double-stranded nucleic acid molecule is siRNA.
<10> A method of treating a renal cancer, the method including administering the combination drug according to any one of <1> to <9> to a subject.
<11> A potentiator for therapeutic effects of a tyrosine kinase inhibitor, the potentiator including:
a dipeptidyl peptidase-4 inhibitor,
wherein the potentiator enhances therapeutic effects of a tyrosine kinase inhibitor on a renal cancer.
<12> The potentiator according to <11>,
wherein the renal cancer is a renal cancer having resistance to the tyrosine kinase inhibitor.
<13> The potentiator according to <11> or <12>,
wherein the tyrosine kinase inhibitor is at least one selected from the group consisting of sunitinib, sorafenib, axitinib, pazopanib, cabozantinib, and lenvatinib.
<14> The potentiator according to any one of <11> to <13>, wherein the dipeptidyl peptidase-4 inhibitor is at least one selected from the group consisting of sitagliptin, linagliptin, alogliptin, teneligliptin, anagliptin, vildagliptin, saxagliptin, trelagliptin, omarigliptin, gemigliptin, evogliptin, gosogliptin, and a dipeptidyl peptidase-4 expression inhibitor.
<15> The potentiator according to <14>,
wherein the dipeptidyl peptidase-4 expression inhibitor is at least one selected from the group consisting of a double-stranded nucleic acid molecule inhibiting expression of a dipeptidyl peptidase-4 gene, DNA including a base sequence encoding the double-stranded nucleic acid molecule, and a vector including the DNA.
<16> The potentiator according to <15>,
wherein the double-stranded nucleic acid molecule inhibiting expression of the dipeptidyl peptidase-4 gene includes:

(a) a sense strand including a base sequence corresponding to a target sequence composed of a base sequence of SEQ ID NO: 1 or SEQ ID NO: 4; and
(b) an antisense strand that constitutes a double stranded nucleic acid with the sense strand of (a), and includes a base sequence complimentary to the sense strand.

<17> The potentiator according <15> or <16>,
wherein the double-stranded nucleic acid molecule is double-stranded RNA or a double-stranded RNA-DNA chimera.
<18> The potentiator according to any one of <15> to <17>, wherein the double-stranded nucleic acid molecule is siRNA or chimeric siRNA.
<19> The potentiator according to any one of <15> to <18>, wherein the double-stranded nucleic acid molecule is

24

siRNA.

<20> A method of enhancing therapeutic effects of a tyrosine kinase inhibitor on a renal cancer, the method including: administering the potentiator according to any one of <11> to <19> to a subject.

**Claims**

1. A combination drug for treating a renal cancer, the combination drug comprising:

   a combination of
   a tyrosine kinase inhibitor, and
   a dipeptidyl peptidase-4 inhibitor.

2. The combination drug according to claim 1,
   wherein the renal cancer is a renal cancer having resistance to the tyrosine kinase inhibitor.

3. The combination drug according to claim 1 or 2,
   wherein the tyrosine kinase inhibitor is at least one selected from the group consisting of sunitinib, sorafenib, axitinib, pazopanib, cabozantinib, and lenvatinib.

4. The combination drug according to any one of claims 1 to 3, wherein the dipeptidyl peptidase-4 inhibitor is at least one selected from the group consisting of sitagliptin, linagliptin, alogliptin, teneligliptin, anagliptin, vildagliptin, saxagliptin, trelagliptin, omarigliptin, gemigliptin, evogliptin, gosogliptin, and a dipeptidyl peptidase-4 expression inhibitor.

5. The combination drug according to claim 4,
   wherein the dipeptidyl peptidase-4 expression inhibitor is at least one selected from the group consisting of a double-stranded nucleic acid molecule inhibiting expression of a dipeptidyl peptidase-4 gene, DNA including a base sequence encoding the double-stranded nucleic acid molecule, and a vector including the DNA.

6. A potentiator for therapeutic effects of a tyrosine kinase inhibitor, the potentiator comprising:
   a dipeptidyl peptidase-4 inhibitor,
   wherein the potentiator enhances therapeutic effects of a tyrosine kinase inhibitor on a renal cancer.

7. The potentiator according to claim 6,
   wherein the renal cancer is a renal cancer having resistance to the tyrosine kinase inhibitor.

8. The potentiator according to claim 6 or 7,
   wherein the tyrosine kinase inhibitor is at least one selected from the group consisting of sunitinib, sorafenib, axitinib, pazopanib, cabozantinib, and lenvatinib.

9. The potentiator according to any one of claims 6 to 8,
   wherein the dipeptidyl peptidase-4 inhibitor is at least one selected from the group consisting of sitagliptin, linagliptin, alogliptin, teneligliptin, anagliptin, vildagliptin, saxagliptin, trelagliptin, omarigliptin, gemigliptin, evogliptin, gosogliptin, and a dipeptidyl peptidase-4 expression inhibitor.

10. The potentiator according to claim 9,
    wherein the dipeptidyl peptidase-4 expression inhibitor is at least one selected from the group consisting of a double-stranded nucleic acid molecule inhibiting expression of a dipeptidyl peptidase-4 gene, DNA including a base sequence encoding the double-stranded nucleic acid molecule, and a vector including the DNA.

FIG.1a

R = 0.057
P = 0.84

FIG.1b

R = 0.66
P = 0.0069

FIG.1c

R = 0.18
P = 0.52

FIG.1d

R = -0.22
P = 0.44

FIG.1e

FIG.1f

FIG.1g

FIG.1h

## FIG.2a

| | PRIMARY TUMOR | | PATIENT-DERIVED SPHEROIDS | | |
|---|---|---|---|---|---|
| | H/E | DPP4 | BRIGHT FIELD | H/E | DPP4 |
| RCC-A | | | | | |
| RCC-B | | | | | |

## FIG.2b

### RCC-A

## FIG.2c

### RCC-B

# FIG.2d

## RCC-A

# FIG.2e

## RCC-B

## FIG.2f

**RCC-A**

FIG.2g

FIG.3a

FIG.3b

FIG.3c

FIG.3d

FIG.3e

FIG.3f

FIG.3g

FIG.3h

FIG.3i

FIG.3j

FIG.3k

FIG.3l

FIG.3m

FIG.3n

FIG.3o

FIG.3p

FIG.4a

FIG.4b

# FIG.4c

## ACHN

# FIG.4d

## ACHN-R

## FIG.4e

### 769-P

## FIG.4f

### 769-P-R

# FIG.4g

## ACHN

# FIG.4h

## ACHN-R

# FIG.4i

# FIG.4j

# FIG.4k

## ACHN-R

# FIG.4l

## 769-P-R

# FIG.4m

## ACHN

# FIG.4n

## 769-P

## ACHN-R

FIG.5a

## 769-P-R

FIG.5b

FIG.5c

Chr2q24: strand(-)

*DPP4*

10 kb

RARE sequence
(-1,324/-1,291 bp

Reference region
(+5,968/+6,149 bp

## ACHN-R

FIG.5d

## 769-P-R

FIG.5e

## ACHN-R

FIG.5f

## 769-P-R

FIG.5g

EP 4 260 873 A1

FIG.5h

293T

FIG.6a

44

FIG.6b

FIG.6c

FIG.6d
ACHN/
SUN

FIG.6e   ACHN-R/ SUN

FIG.6f   ACHN-R/ SUN +SITA

FIG.6g

# FIG.6h

Legend:
- ACHN/SUN
- **ACHN-R/**SUN
- **ACHN-R/**SUN+SI

# FIG.6i

Legend:
- ACHN/SUN
- **ACHN-R/**SUN
- **ACHN-R/**SUN+SI

# FIG.7a

FIG.7b

ALL CASES
(n = 73)

☐ T2DM−/DPP4i− (n = 47)
▨ T2DM+/DPP4i− (n = 12)
■ T2DM+/DPP4i+ (n = 14)

MAXIMUM TUMOR CHANGE

(%)
100
50
0
-50
-100

## FIG.7c

## FIG.7d

# FIG.7e

# FIG.7f

FIG.7g

## INTERNATIONAL SEARCH REPORT

| International application No. |
| --- |
| **PCT/JP2021/045772** |

### A. CLASSIFICATION OF SUBJECT MATTER

*A61K 45/06*(2006.01)i; *A61K 31/40*(2006.01)i; *A61K 31/403*(2006.01)i; *A61K 31/404*(2006.01)i; *A61K 31/4162*(2006.01)i; *A61K 31/44*(2006.01)i; *A61K 31/4439*(2006.01)i; *A61K 31/47*(2006.01)i; *A61K 31/496*(2006.01)i; *A61K 31/4985*(2006.01)i; *A61K 31/506*(2006.01)i; *A61K 31/513*(2006.01)i; *A61K 31/519*(2006.01)i; *A61K 31/522*(2006.01)i; *A61K 31/713*(2006.01)i; *A61K 48/00*(2006.01)i; *A61P 35/00*(2006.01)i; *A61P 43/00*(2006.01)i; *C12N 9/99*(2006.01)i; *C12N 15/11*(2006.01)i; *C12N 15/113*(2010.01)i

FI: A61K45/06; A61K31/40; A61K31/403; A61K31/404; A61K31/4162; A61K31/44; A61K31/4439; A61K31/47; A61K31/496; A61K31/4985; A61K31/506; A61K31/513; A61K31/519; A61K31/522; A61K31/713; A61K48/00; A61P35/00 ZNA; A61P43/00 121; C12N9/99; C12N15/11 Z; C12N15/113 Z

According to International Patent Classification (IPC) or to both national classification and IPC

### B. FIELDS SEARCHED

Minimum documentation searched (classification system followed by classification symbols)

A61K45/06; A61K31/40; A61K31/403; A61K31/404; A61K31/4162; A61K31/44; A61K31/4439; A61K31/47; A61K31/496; A61K31/4985; A61K31/506; A61K31/513; A61K31/519; A61K31/522; A61K31/713; A61K48/00; A61P35/00; A61P43/00; C12N9/99; C12N15/11; C12N15/113

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2019-530671 A (TR. OF TUFTS COLLEGE) 24 October 2019 (2019-10-24) paragraphs [0087], [0094], [0354] | 1, 3-6, 8-10 |
| A | | 2, 7 |
| Y | 大家基嗣, 低分子阻害剤による治療の実践-4 ソラフェニブ, スニチニブ薬理作用と治療の実際, 薬局, 2010, vol. 61, no. 2, pages 73-80, (The Journal of Practical Pharmacy.), non-official translation (OYA, Mototsugu. Practice of treatment with small molecule inhibitors-4. Sorafenib, sunitinib pharmacological action and treatment practice.) page 74, left column, second paragraph, page 75, right column, third paragraph to page 79, left column, first paragraph | 1, 3-6, 8-10 |
| A | | 2, 7 |

☑ Further documents are listed in the continuation of Box C.　　☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **01 February 2022** | **15 February 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 260 873 A1**

**INTERNATIONAL SEARCH REPORT**

International application No.

**PCT/JP2021/045772**

**C.      DOCUMENTS CONSIDERED TO BE RELEVANT**

| Category* | Citation of document, with indication, where appropriate, of the relevant passages | Relevant to claim No. |
|---|---|---|
| Y | ALMAGTHALI, A. G. et al. Dipeptidyl peptidase-4 inhibitors: Anti-diabetic drugs with potential effects on cancer. Diabetes Metab. Syndr. 2019, vol. 13, no. 1, pages 36-39<br>abstract, page 6211, left column, second paragraph to right column, first paragraph | 1, 3-6, 8-10 |
| A |  | 2, 7 |
| Y | LI, Y. L. et al. ApoC1 promotes the metastasis of clear cell renal cell carcinoma via activation of STAT3. Oncogene. September 2020, vol. 39, no. 39, pages 6203-6217<br>page 37, right column, second paragraph, page 38, left column, fifth paragraph | 1, 3-6, 8-10 |
| A |  | 2, 7 |
| P, X | KAMADA, S. et al. Functional inhibition of cancer stemness-related protein DPP4 rescues tyrosine kinase inhibitor resistance in renal cell carcinoma. Oncogene. June 2021, vol. 40, no. 22, pages 3899-3913<br>abstract, results, materials and methods | 1-10 |

Form PCT/ISA/210 (second sheet) (January 2015)

54

**INTERNATIONAL SEARCH REPORT**

International application No.

**PCT/JP2021/045772**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

International application No.

**PCT/JP2021/045772**

| Patent document cited in search report | | | Publication date (day/month/year) | Patent family member(s) | | | Publication date (day/month/year) |
|---|---|---|---|---|---|---|---|
| JP | 2019-530671 | A | 24 October 2019 | US | 2019/0209525 | A1 | |
| | | | | paragraphs [0186], [0193], [1504] | | | |
| | | | | WO | 2018/049027 | A1 | |
| | | | | EP | 3509604 | A1 | |
| | | | | KR | 10-2019-0045311 | A | |
| | | | | CN | 109906082 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SIEGEL, R. L. ; MILLER, K. D. ; JEMAL, A.** Cancer statistics. *CA Cancer J. Clin.,* 2018, vol. 68, 7-30
- **BRAY, F.** Global cancer statistics 2018: GLOBOCAN estimates of incidence and mortality worldwide for 36 cancers in 185 countries. *CA Cancer J. Clin.,* 2018, vol. 68, 394-424
- **PLIMACK, E. R.** Checkpoint Inhibitors for the Treatment of Renal Cell Carcinoma. *Current treatment options in oncology,* 2017, vol. 18, 7
- **CLEVERS, H.** The cancer stem cell: premises, promises and challenges. *Nat. Med.,* 2011, vol. 17, 313-319
- **ISHIBASHI, K. et al.** Overriding TKI resistance of renal cell carcinoma by combination therapy with IL-6 receptor blockade. *Oncotarget,* 2017, vol. 8, 55230-55245
- **EISENHAUER, E. A. et al.** New response evaluation criteria in solid tumours: revised RECIST guideline. *Eur. J. Cancer,* 2009, vol. 45, 228-247

- **NAMEKAWA, T et al.** ALDH1A1 in patient-derived bladder cancer spheroids activates retinoic acid signaling leading to TUBB3 overexpression and tumor progression. *Int. J. Cancer,* 2019, vol. 146, 1099-1113
- **WASSERMAN W. W. ; SANDELIN A.** Applied bioinformatics for the identification of regulatory elements. *Nat. Rev. Genet.,* 2004, vol. 5, 276-287
- **BULENS, F. et al.** Retinoic acid induction of human tissue-type plasminogen activator gene expression via a direct repeat element (DR5) located at -7 kilobases. *J. Biol. Chem.,* 1995, vol. 270, 7167-7175
- **STANY, M. P. et al.** Identification of novel therapeutic targets in microdissected clear cell ovarian cancers. *PLoS One,* 2011, vol. 6, e21121
- **WASSERMAN W. W. ; SANDELIN A.** Applied bioinformatics for the identification of regulatory elements. *Nat. Rev. Genet.,* 2004, vol. 5, 276-287